# EUROPÄISCHE PATENTSCHRIFT

(11) **EP 2 646 418 B1**
(45) Veröffentlichungstag und Bekanntmachung des Hinweises auf die Patenterteilung: **04.10.2017**
(21) Anmeldenummer: 11794438.9
(22) Anmeldetag: 28.11.2011
(51) Int. Cl.: C07D 239/26, A01N 43/54

(54) **PYRIMIDIN-DERIVATE UND IHRE VERWENDUNG ALS SCHÄDLINGSBEKÄMPFUNGSMITTEL**
PYRIMIDINE DERIVATIVES AND THEIR USE AS PARASITIC AGENTS
DÉRIVÉS DE PYRIMIDINE ET LEUR UTILISATION COMME AGENTS PARASITICIDES.

(30) Priorität: 30.11.2010 EP 10193115; 01.12.2010 US 418526 P
(43) Veröffentlichungstag der Anmeldung: 09.10.2013
(73) Patentinhaber: Bayer Intellectual Property GmbH, 40789 Monheim am Rhein (DE)
(72) Erfinder: NISING, Carl Friedrich, 51381 Leverkusen (DE); HOLMWOOD, Graham, 51371 Leverkusen (DE); HELMKE, Hendrik, 65835 Liederbach (DE); PERIS, Gorka, 50737 Köln (DE); TSUCHIYA, Tomoki, 69009 Lyon (FR); SUDAU, Alexander, 40764 Langenfeld (DE); BENTING, Jürgen, 42799 Leichlingen (DE); DAHMEN, Peter, 41470 Neuss (DE); WACHENDORFF-NEUMANN, Ulrike, 56566 Neuwied (DE)
(74) Vertreter: BIP Patents
(86) Internationale Anmeldenummer: PCT/EP2011/071123
(87) Internationale Veröffentlichungsnummer: WO 2012/072547

(56) Entgegenhaltungen:
- EP-A1- 0 221 014
- EP-A2- 0 131 867
- EP-A2- 0 316 663
- WO-A2-99/26927
- DE-A1- 2 428 372
- US-A- 4 002 628

## Beschreibung

Die vorliegende Erfindung betrifft neue substituierte Pyrimidin-Derivate, Verfahren zum Herstellen dieser Verbindungen, Mittel enthaltend diese Verbindungen, sowie deren Verwendung als biologisch aktive Verbindungen, insbesondere zur Bekämpfung von schädlichen Mikroorganismen im Pflanzenschutz und im Materialschutz und als Pflanzenwachstumsregulatoren.

Es ist bereits bekannt, dass bestimmte Pyrimidin-Derivate im Pflanzenschutz als Fungizide und/oder Wachstumsregulatoren eingesetzt werden können (vgl. EP-A 0 001 399, EP-A 0 028 755, EP-A 0 316 663, EP-A 0 131 867). Da sich die ökologischen und ökonomischen Anforderungen an moderne Wirkstoffe, z.B. Fungizide, laufend erhöhen, beispielsweise bezüglich Wirkspektrum, Toxizität, Selektivität, Aufwandmenge, Rückstandsbildung und günstige Herstellbarkeit, und außerdem z.B. Probleme mit Resistenzen auftreten können, besteht die ständige Notwendigkeit, neue fungizide Mittel zu entwickeln, die zumindest in Teilbereichen Vorteile gegenüber den bekannten aufweisen.

Es wurden nun neue substituierte Pyrimidin-Derivate der Formel (I) 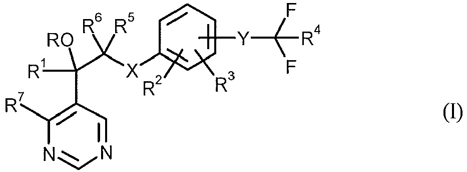 gefunden, in welcher
- X: für O, S, SO, SO₂, CH₂ oder für eine direkte Bindung steht,
- R: für Wasserstoff, Alkyl, Tri(C₁-C₃-alkyl)silyl, Formyl oder Acetyl steht,
- R¹: für *tert*-Butyl, 1-Propinyl, 1-Chlorcyclopropyl, 1-Fluorcyclopropyl, 1-Methylcyclopropyl oder 2,4-Difluorphenyl steht,
- R² und R³: jeweils für Wasserstoff stehen,
- Y: für O, S, SO oder SO₂ steht,
- R⁴: für Wasserstoff, Fluor, Chlor oder C₁-C₄-Halogenalkyl steht,
- R⁵ und R⁶: gleich oder verschieden sind und jeweils für Wasserstoff, Halogen oder gegebenenfalls substituiertes Alkyl stehen, oder gemeinsam für die Gruppe -CH₂-CH₂- stehen, sodass mit dem Kohlenstoffatom, an welches sie gebunden sind, ein Cyclopropyl-Ring entsteht,
- R⁷: für Wasserstoff, Halogen, C₁-C₄-Alkyl oder C₁-C₄-Halogenalkyl steht,
sowie deren agrochemisch wirksamen Salze.

Die so erhältlichen Salze weisen ebenfalls fungizide und/oder pflanzenwachstumsregulatorische Eigenschaften auf.

Die erfindungsgemäßen verwendbaren Pyrimidin-Derivate können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die Verbindungen der Formel (I) liegen insbesondere gegebenenfalls in Form von Enantiomeren vor:

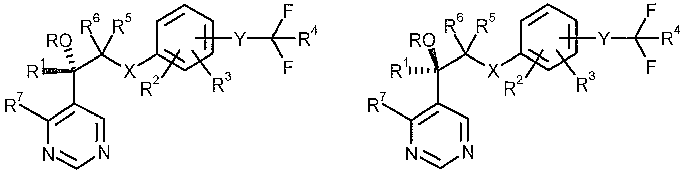

Sind die Substituenten R⁵ und R⁶ verschieden, liegen folgende Diastereomere gegebenenfalls in unterschiedlichsten Gemischen vor:

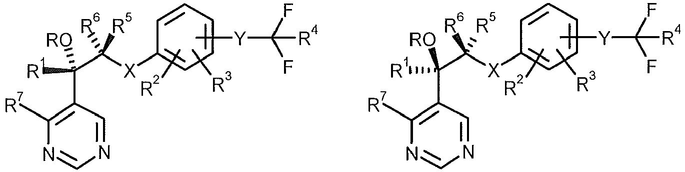

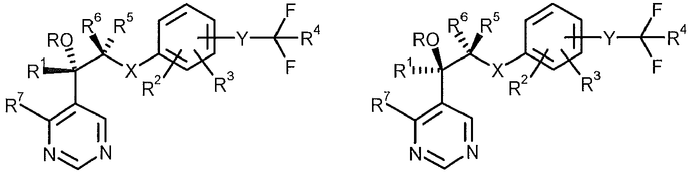

Die erfindungsgemäß verwendbaren Pyrimidin-Derivate sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte (siehe auch unten unter "Erläuterungen der Verfahren und Zwischenprodukte") gleichermaßen.
- X: steht bevorzugt für O, S, CH₂ oder für eine direkte Bindung.
- X: steht besonders bevorzugt für O, S oder CH₂.
- X: steht ganz besonders bevorzugt für O.
- X: steht auch ganz besonders bevorzugt für CH₂.

- R: steht bevorzugt für Wasserstoff, Methyl, Trimethylsilyl, Formyl oder Acetyl.
- R: steht besonders bevorzugt für Wasserstoff.

- R¹: steht für *tert*-Butyl, 1-Propinyl, 1-Chlorcyclopropyl, 1-Fluorcyclopropyl, 1-Methylcyclopropyl, 2,4-Difluorphenyl.

- R² und R³: stehen für Wasserstoff.
- Y: steht bevorzugt für O oder S.
- Y: steht besonders bevorzugt für O.
- Y: steht auch besonders bevorzugt für S.

- R⁴: steht bevorzugt für Wasserstoff, Fluor, Chlor oder C₁-C₂-Halogenalkyl.
- R⁴: steht besonders bevorzugt für Wasserstoff, Fluor, Chlor, Difluormethyl, Trifluormethyl oder Difluorchlormethyl.
- R⁴: steht ganz besonders bevorzugt für Wasserstoff, Fluor oder Chlor.

- R⁵ und R⁶: sind gleich oder verschieden und stehen bevorzugt jeweils für Wasserstoff, Fluor, Chlor, Brom, Iod, C₁-C₄-Alkyl oder C₁-C₄-Halogenalkyl, oder gemeinsam für die Gruppe -CH₂-CH₂-.
- R⁵ und R⁶: sind gleich oder verschieden und stehen besonders bevorzugt jeweils für Wasserstoff, Fluor, Chlor, Methyl, Ethyl oder Trifluormethyl, oder gemeinsam für die Gruppe -CH₂-CH₂-.
- R⁵ und R⁶: sind gleich oder verschieden und stehen ganz besonders bevorzugt jeweils für Wasserstoff oder Methyl, oder gemeinsam für die Gruppe -CH₂-CH₂-.

- R⁷: steht bevorzugt für Wasserstoff, Fluor, Chlor, Brom, C₁-C₄-Alkyl oder C₁-C₂-Halogenalkyl.
- R⁷: steht besonders bevorzugt für Wasserstoff, Fluor, Chlor, Methyl, Ethyl, n-Propyl, i-Propyol, Difluormethyl, Trifluormethyl oder Difluorchlormethyl.
- R⁷: steht ganz besonders bevorzugt für Wasserstoff, Chlor, Methyl, Difluormethyl, Trifluormethyl oder Difluorchlormethyl.

Eine weitere Ausführungsform der vorliegenden Erfindung betrifft Verbindungen der Formel (I), in welcher R, R² und R³ gleichzeitig jeweils für Wasserstoff stehen.
Eine weitere Ausführungsform der vorliegenden Erfindung betrifft Verbindungen der Formel (I), in welcher R, R² und R³ gleichzeitig jeweils für Wasserstoff stehen und R⁴ für Fluor steht.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.
Bevorzugt sind solche Verbindungen der Formel (I), in welcher alle Reste jeweils die oben genannten bevorzugten Bedeutungen haben.
Besonders bevorzugt sind solche Verbindungen der Formel (I), in welcher alle Reste jeweils die oben genannten besonders bevorzugten Bedeutungen haben.

### Erläuterung der Verfahren und Zwischenprodukte

Die Pyrimidin-Derivate der Formel (I) lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

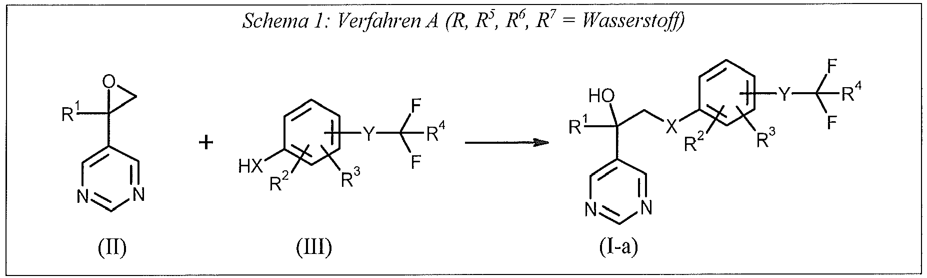

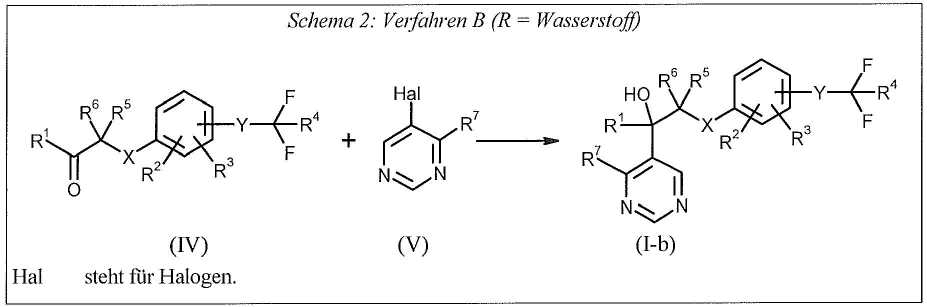

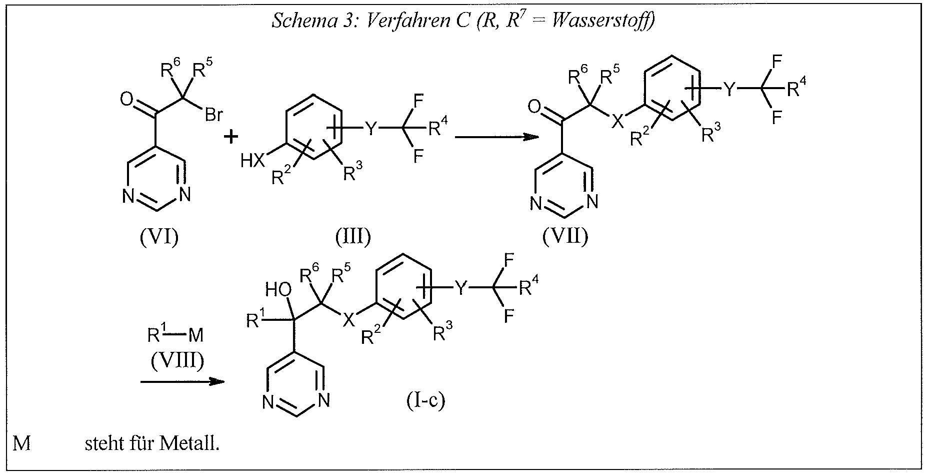

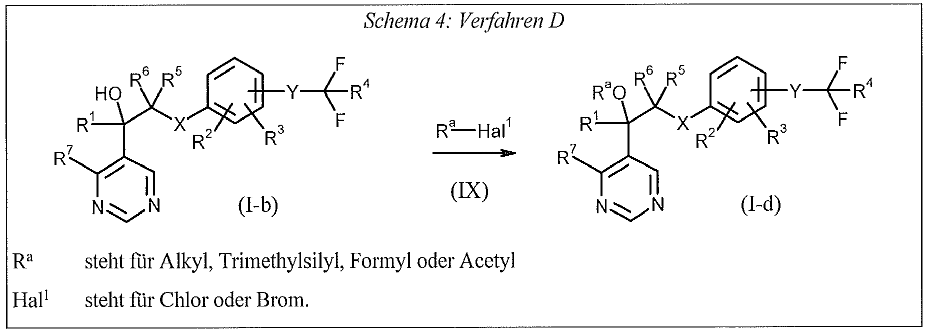

Bevorzugte Restedefinitionen der vorstehenden und nachfolgend genannten Formeln und Schemata sind bereits oben angegeben. Diese Definitionen gelten nicht nur für die Endprodukte der Formel (I) sondern auch für alle Zwischenprodukte gleichermaßen.

### Verfahren A

Die bei der Durchführung des erfindungsgemäßen Verfahrens A als Ausgangstoffe benötigten Oxiran-Derivate der Formel (II) sind teilweise bekannt und lassen sich nach bekannten Verfahren herstellen (vgl. DE-A 31 11 238 und EP-A 0 157 712).

Neu und ebenfalls Gegenstand dieser Anmeldung sind Oxiran-Derivate der Formel (II-a) 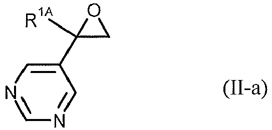 in welcher
- R^{1A}: für 1-Propinyl oder 2,4-Difluorphenyl steht.

Die (Thio)phenole der Formel (III) sind bekannt oder lassen sich nach bekannten Verfahren erhalten.

Das erfindungsgemäße Verfahren A wird in Gegenwart eines Verdünnungsmittels und gegebenenfalls in Gegenwart einer Base durchgeführt. Gegebenenfalls wird an die erhaltene Verbindung der Formel (I-a) anschließend eine Säure oder ein Metallsalz addiert (siehe unten).

Als Verdünnungsmittel kommen für die erfindungsgemäße Umsetzung alle inerten organischen Lösungsmittel infrage. Hierzu gehören vorzugsweise Alkohole, wie z.B. Ethanol und Methoxyethanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle üblicherweise verwendbaren organischen und anorganischen Basen in Betracht. Hierzu gehören vorzugsweise Alkalicarbonate, wie z.B. Natrium- oder Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin. Besonders bevorzugt verwendet man Natriumhydrid.

Die Reaktionstemperaturen können bei der Durchführung des erfindungsgemäßen Verfahrens A in einem größeren Bereich variiert werden. Im Allgemeinen arbeitet man bei Temperaturen zwischen 0 °C und 200 °C, vorzugsweise zwischen 60 °C und 150 °C.

Die erfindungsgemäße Umsetzung kann gegebenenfalls unter erhöhtem Druck durchgeführt werden. Im Allgemeinen arbeitet man zwischen 1 und 50 bar, vorzugsweise zwischen 1 und 25 bar.

Bei der Durchführung des erfindungsgemäßen Verfahrens A setzt man auf 1 Mol Oxiran der allgemeinen Formel (II) vorzugsweise 1 bis 2 Mol (Thio)phenol der Formel (III) und gegebenenfalls 1 bis 2 Mol Base ein. Die Isolierung der Endprodukte erfolgt in allgemein üblicher Weise.

### Verfahren B

Die bei der Durchführung des erfindungsgemäßen Verfahrens B als Ausgangstoffe benötigten Ketone der Formel (IV) sind teilweise bekannt.

Neu und damit ebenfalls Gegenstand dieser Erfindung sind Ketone der Formel (IV-a) 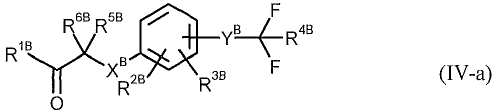 in welcher
- X^{B}: für O steht,
- R^{1B}: für *tert*-Butyl, 1-Propinyl, 1-Chlorcyclopropyl, 1-Fluorcyclopropyl oder 1-Methylcyclopropylsteht,
- R^{2B} und R^{3B}: jeweils für Wasserstoff stehen,
- Y^{B}: für O, S, SO oder SO₂ steht,
- R^{4B}: für Wasserstoff, Fluor, Chlor oder C₁-C₄-Halogenalkyl steht,
- R^{5B} und R^{6B}: gleich oder verschieden sind und jeweils Wasserstoff, Halogen oder gegebenenfalls substituiertes Alkyl stehen, oder gemeinsam für die Gruppe -CH₂-CH₂- stehen, sodass mit dem Kohlenstoffatom, an welches sie gebunden sind, ein Cyclopropyl-Ring entsteht.

- X^{B}: steht bevorzugt für O.

- Y^{B}: steht bevorzugt für O oder S.
- Y^{B}: steht besonders bevorzugt für O.
- Y^{B}: steht auch besonders bevorzugt für S.

- R^{4B}: steht bevorzugt für Wasserstoff, Fluor, Chlor oder C₁-C₂-Halogenalkyl.
- R^{4B}: steht besonders bevorzugt für Wasserstoff, Fluor, Chlor, Difluormethyl, Trifluormethyl oder Difluorchlormethyl.
- R^{4B}: steht ganz besonders bevorzugt für Wasserstoff, Fluor oder Chlor.

- R^{5B} und R^{6B}: sind gleich oder verschieden und stehen bevorzugt jeweils für Wasserstoff, Fluor, Chlor, Brom, Iod, C₁-C₄-Alkyl oder C₁-C₄-Halogenalkyl, oder gemeinsam für die Gruppe -CH₂-CH₂-.
- R⁵ und R⁶: sind gleich oder verschieden und stehen besonders bevorzugt jeweils für Wasserstoff, Fluor, Chlor, Methyl, Ethyl oder Trifluormethyl, oder gemeinsam für die Gruppe -CH₂-CH₂-.
- R^{5B} und R^{6B}: sind gleich oder verschieden und stehen ganz besonders bevorzugt jeweils für Wasserstoff oder Methyl, oder gemeinsam für die Gruppe -CH₂-CH₂-.

Neu und damit ebenfalls Gegenstand dieser Erfindung sind Ketone der Formel (IV-b) 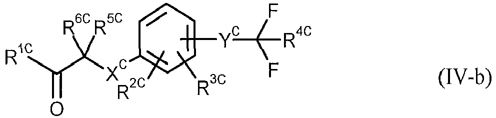 in welcher
- X^{C}: für S, SO, SO₂ steht,
- R^{1C}: für 1-Propinyl, 1-Chlorcyclopropyl, 1-Fluorcyclopropyl oder 1-Methylcyclopropyl steht,
- R^{2C} und R^{3C}: jeweils für Wasserstoff stehen,
- Y^{C}: für O, S, SO oder SO₂ steht,
- R^{4C}: für Wasserstoff, Fluor, Chlor oder C₁-C₄-Halogenalkyl steht,
- R^{5C} und R^{6C}: gleich oder verschieden sind und jeweils Wasserstoff, Halogen oder gegebenenfalls substituiertes Alkyl stehen, oder gemeinsam für die Gruppe -CH₂-CH₂- stehen, sodass mit dem Kohlenstoffatom, an welches sie gebunden sind, ein Cyclopropyl-Ring entsteht.

- X^{C}: steht bevorzugt für S.

- Y^{C}: steht bevorzugt für O oder S.
- Y^{C}: steht besonders bevorzugt für O.
- Y^{C}: steht auch besonders bevorzugt für S.

- R^{4C}: steht bevorzugt für Wasserstoff, Fluor, Chlor oder C₁-C₂-Halogenalkyl.
- R^{4C}: steht besonders bevorzugt für Wasserstoff, Fluor, Chlor, Difluormethyl, Trifluormethyl oder Difluorchlormethyl.
- R^{4C}: steht ganz besonders bevorzugt für Wasserstoff, Fluor oder Chlor.

- R^{5C} und R^{6C}: sind gleich oder verschieden und stehen bevorzugt jeweils für Wasserstoff, Fluor, Chlor, Brom, Iod, C₁-C₄-Alkyl oder C₁-C₄-Halogenalkyl, oder gemeinsam für die Gruppe -CH₂-CH₂-.
- R^{5C} und R^{6C}: sind gleich oder verschieden und stehen besonders bevorzugt jeweils für Wasserstoff, Fluor, Chlor, Methyl, Ethyl oder Trifluormethyl, oder gemeinsam für die Gruppe -CH₂-CH₂-.
- R^{5C} und R^{6C}: sind gleich oder verschieden und stehen ganz besonders bevorzugt jeweils für Wasserstoff oder Methyl, oder gemeinsam für die Gruppe -CH₂-CH₂-.

Ketone der Formel (IV) lassen sich auf bekannte Weise herstellen (vgl. EP-A 0 040 345, EP-A 0 001 399). Ketone der Formel (IV) werden beispielsweise nach folgendem Verfahren erhalten:

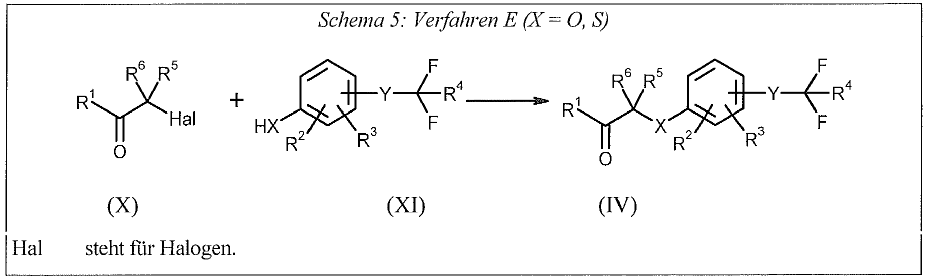

Die Halogenide der Formel (X) sind bekannt. In Formel (X) steht Hal bevorzugt für Chlor oder Brom.

Das erfindungsgemäße Verfahren E wird in Gegenwart eines Verdünnungsmittels und in Gegenwart einer anorganischen Base durchgeführt.

Als Verdünnungsmittel kommen für die erfindungsgemäße Umsetzung alle inerten organischen Lösungsmittel infrage. Hierzu gehören vorzugsweise Ketone, wie z.B. Aceton oder 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder chlorierte Kohlenwasserstoffe, wie z.B. Dichlormethan.

Als Basen kommen für die erfindungsgemäße Umsetzung alle üblicherweise verwendbaren organischen und anorganischen Basen in Betracht. Hierzu gehören vorzugsweise Alkalicarbonate, wie z.B. Natrium- oder Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin. Besonders bevorzugt verwendet man Natriumhydrid.

Die Reaktionstemperaturen können beim erfindungsgemäßen Verfahren B in einem bestimmten Bereich variiert werden. Im Allgemeinen arbeitet man zwischen 50 °C und 150 °C, vorzugsweise zwischen 20 °C und 100 °C.

Die erfindungsgemäße Umsetzung wird vorzugsweise unter Inertgas, wie insbesondere Stickstoff oder Argon, vorgenommen.

Bei der Durchführung des erfindungsgemäßen Verfahrens E setzt man die Halogenide der Formel (X) und die (Thio)alkohole der Formel (XI) in etwa äquimolaren Mengen ein, Unter- oder Überschreitungen um bis zu ca. 20 Molprozent sind jedoch möglich. Die anorganische Base wird vorteilhaft im Überschuss von 5 bis 75 Molprozent, vorzugsweise von 10 bis 50 Molprozent, verwendet.

Die bei der Durchführung des erfindungsgemäßen Verfahrens B als Ausgangstoffe ebenfalls benötigten Pyrimidinylhalogenide der Formel (V) sind bekannt.

Das erfindungsgemäße Verfahren B wird in Gegenwart eines Verdünnungsmittels und in Gegenwart einer alkalimetall-organischen Verbindung durchgeführt. Gegebenenfalls wird an die erhaltene Verbindung der Formel (I-b) anschließend eine Säure oder ein Metallsalz addiert (siehe unten).

Für die erfindungsgemäße Umsetzung kommen als Verdünnungsmittel vorzugsweise inerte organische Lösungsmittel in Frage. Hierzu gehören vorzugsweise solche, die einen tiefen Festpunkt besitzen, wie insbesondere Ether, wie Diethylether oder Tetrahydrofuran. Vorzugsweise arbeitet man mit Mischungen aus diesen beiden Ethern.

Als alkalimetall-organische Verbindungen werden bei der erfindungsgemäßen Umsetzung vorzugsweise Alkalimetall-alkyle, wie insbesondere n-Butyl-Lithium, eingesetzt; es können aber auch Alkalimetallaryle, wie Phenyl-Lithium, Verwendung finden.

Die Reaktionstemperaturen können beim erfindungsgemäßen Verfahren in einem bestimmten Bereich variiert werden. Im Allgemeinen arbeitet man zwischen-150 °C und -50 °C, vorzugsweise zwischen -120 °C und -80 °C.

Die erfindungsgemäße Umsetzung wird vorzugsweise unter Inertgas, wie insbesondere Stickstoff oder Argon, vorgenommen.

Bei der Durchführung des erfindungsgemäßen Verfahrens setzt man die Ketone der Formel (IV) und die Halogenide der Formel (V) in etwa äquimolaren Mengen ein, Unter- oder Überschreitungen um bis zu ca. 20 Molprozent sind jedoch möglich. Die alkalimetall-organische Verbindung wird vorteilhaft im Überschuss von 5 bis 75 Molprozent, vorzugsweise von 10 bis 50 Molprozent, verwendet.

Es kann dabei so verfahren werden, dass man zunächst die alkalimetall-organische Verbindung mit dem Halogenid der Formel (V) reagieren lässt und anschließend die Keto-Verbindung der Formel (IV) zufügt; man kann aber auch die Keto-Verbindung und das Halogenid vorlegen und anschließend bei tiefer Temperatur (z.B. bei -100 °C bis -130 °C) die alkalimetall-organische Verbindung zugeben. Die Isolierung der Verbindungen der Formel (I-b) erfolgt in der Weise, dass das bei der Reaktion zunächst gebildete Alkalialkanolat (z.B. Lithium-alkanolat) mit Wasser hydrolysiert wird. Die weitere Aufarbeitung erfolgt dann in üblicher Weise.

### Verfahren C

Die Bromide der Formel (VI) sind teilweise bekannt. Neu und damit ebenfalls Gegenstand dieser Erfindung sind Bromide der Formel (VI-a) 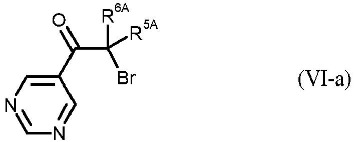 in welcher
- R^{5A}: für Halogen oder substituiertes Alkyl steht,
- R^{6A}: für Halogen oder substituiertes Alkyl steht.

- R^{5A} und R^{6A}: sind gleich oder verschieden und stehen bevorzugt jeweils für Fluor, Chlor, Brom, Iod oder C₁-C₄-Halogenalkyl, oder gemeinsam für die Gruppe -CH₂-CH₂-.
- R^{5A} und R^{6A}: sind gleich oder verschieden und stehen besonders bevorzugt jeweils für Fluor, Chlor oder Trifluormethyl, oder gemeinsam für die Gruppe -CH₂-CH₂-.
- R^{5A} und R^{6A}: sind gleich oder verschieden und stehen ganz besonders bevorzugt gemeinsam für die Gruppe -CH₂-CH₂-.

Die (Thio)phenole der Formel (III) sind ebenfalls bekannt (siehe oben bei Verfahren A).

Die bei der Durchführung des erfindungsgemäßen Verfahrens C als Zwischenprodukte auftretenden Ketone der Formel (VII) sind teilweise bekannt.

Die Organometallverbindungen der Formel (VIII) sind bekannt, wobei M in Formel (VIII) vorzugsweise für Lithium oder Magnesium steht.

Das erfindungsgemäße Verfahren C (Schritt 1) wird in Gegenwart eines Verdünnungsmittels und gegebenenfalls in Gegenwart einer Base durchgeführt. Als Verdünnungsmittel kommen für die erfindungsgemäße Umsetzung alle inerten organischen Lösungsmittel infrage. Hierzu gehören vorzugsweise Alkohole, wie z.B. Ethanol und Methoxyethanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. o Benzol und Toluol; Amide, wie z.B. Dimethylformamid: oder Sulfoxide, wie z.B. Dimethylsulfoxid.

Als Basen kommen für die erfindungsgemäße Umsetzung alle üblicherweise verwendbaren organischen und anorganischen Basen in Betracht. Hierzu gehören vorzugsweise Alkalicarbonate, wie z.B. Natrium- oder Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens C in einem größeren Bereich variiert werden. Im Allgemeinen arbeitet man bei Temperaturen zwischen 0 °C und 200 °C, vorzugsweise zwischen 20 °C und 100 °C.

Die erfindungsgemäße Umsetzung kann gegebenenfalls unter erhöhtem Druck durchgeführt werden. Im Allgemeinen arbeitet man zwischen 1 und 50 bar, vorzugsweise zwischen 1 und 25 bar.

Bei der Durchführung des erfindungsgemäßen Verfahrens C (Schritt 1) setzt man auf 75 bis 112 mMol Bromketon der allgemeinen Formel (VI) vorzugsweise 25m Mol (Thio)Phenol der Formel (III) und gegebenenfalls 75 bis 112 mMol Base ein, in Dimethylsulfoxid als Lösungsmittel. Die Isolierung der Endprodukte erfolgt in allgemein üblicher Weise.

Das erfindungsgemäße Verfahren C (Schritt 2) wird in Gegenwart eines Verdünnungsmittels und in Gegenwart einer metall-organischen Verbindung durchgeführt. Gegebenenfalls wird an die erhaltene Verbindung der Formel (I-c) anschließend eine Säure oder ein Metallsalz addiert (siehe unten).

Für die erfindungsgemäße Umsetzung von Verbindungen der Formel (VII) zu Verbindungen der Formel (I-c) kommen als Verdünnungsmittel vorzugsweise inerte organische Lösungsmittel in Frage. Hierzu gehören insbesondere Ether, wie Diethylether oder Tetrahydrofuran; aromatische Kohlenwasserstoffe, wie z.B. Benzol und Toluol. Als metall-organische Verbindungen werden bei der erfindungsgemäßen Umsetzung vorzugsweise Erdalkalimetall-alkyle, wie insbesondere Methyl-Magnesiumbromid, eingesetzt; es können aber auch Alkalimetall-alkyle, wie n-Butyllithium, Verwendung finden.

Die Reaktionstemperaturen können beim erfindungsgemäßen Verfahren in einem bestimmten Bereich variiert werden. Im Allgemeinen arbeitet man zwischen -100 °C und +20 °C, vorzugsweise zwischen -78 °C und 0 °C.

Die erfindungsgemäße Umsetzung wird vorzugsweise unter Inertgas, wie insbesondere Stickstoff oder Argon, vorgenommen.

Bei der Durchführung des erfindungsgemäßen Verfahrens setzt man 2.5 mMol Keton der Formel (VII) mit den Organometallverbindungen der Formel (VIII) im Überschuss von 300 Molprozent in Toluol um. Die weitere Aufarbeitung erfolgt dann in üblicher Weise.

Es kann dabei so verfahren werden, dass man zunächst das Keton (VII) vorlegt und anschließend bei geeigneter Temperatur (z. B. 0°C) die Organometallverbindung der Formel (VIII) zufügt. Die Isolierung der Verbindungen der Formel (I-c) erfolgt in der Weise, dass das bei der Reaktion zunächst gebildete Metallalkanolat (z.B. Magnesium-alkanolat) mit Wasser hydrolysiert wird. Die weitere Aufarbeitung erfolgt dann in üblicher Weise.

### Verfahren D

Die bei der Durchführung des erfindungsgemäßen Verfahrens D als Ausgangstoffe benötigten Alkohol-Derivate der Formel (I-b) sind Gegenstand dieser Erfindung und lassen sich gemäß den Verfahren A bis C herstellen.

Die Halogenide der Formel (IX) sind bekannt.

Das erfindungsgemäße Verfahren D wird in Gegenwart eines Verdünnungsmittels und gegebenenfalls in Gegenwart einer Base durchgeführt. Gegebenenfalls wird an die erhaltene Verbindung der Formel (I-b) anschließend eine Säure oder ein Metallsalz addiert (siehe unten).

Als Verdünnungsmittel kommen für die erfindungsgemäße Umsetzung alle inerten organischen Lösungsmittel infrage. Hierzu gehören vorzugsweise Ketone, wie z.B. Aceton oder 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder chlorierte Kohlenwasserstoffe, wie z.B. Dichlormethan.

Als Basen kommen für die erfindungsgemäße Umsetzung alle üblicherweise verwendbaren organischen und anorganischen Basen in Betracht. Hierzu gehören vorzugsweise Alkalicarbonate, wie z.B. Natrium- oder Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin. Besonders bevorzugt verwendet man Natriumhydrid.

Die Reaktionstemperaturen können bei der Durchführung des erfindungsgemäßen Verfahrens D in einem größeren Bereich variiert werden. Im Allgemeinen arbeitet man bei Temperaturen zwischen -20 °C und 100 °C, vorzugsweise zwischen 0 °C und 60 °C.

Die erfindungsgemäße Umsetzung kann gegebenenfalls unter erhöhtem Druck durchgeführt werden. Im Allgemeinen arbeitet man zwischen 1 und 50 bar, vorzugsweise zwischen 1 und 25 bar.

Bei der Durchführung des erfindungsgemäßen Verfahrens D setzt man auf 1 Mol Alkohol der allgemeinen Formel (I-b) vorzugsweise 1 bis 2 Mol Halogenid der Formel (IX) und gegebenenfalls 1 bis 2 Mol Base ein. Die Isolierung der Endprodukte erfolgt in allgemein üblicher Weise.

Die nach den erfindungsgemäßen Verfahren erhältlichen Verbindungen der allgemeinen Formel (I) können in Säureadditions-Salze bzw. Metallsalz-Komplexe überführt werden.

Zur Herstellung von physiologisch verträglichen Säureadditionssalzen der Verbindungen der allgemeinen Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure sowie Sulfonsäuren, wie z.B. p-Toluolsulfonsäure und 1,5-Naphthalindisulfonsäure.

Die Säureadditions-Salze der Verbindungen der all-gemeinen Formel (I) können in einfacher Weise nach üblichen Salzbildungsmethoden, z.B. durch Lösung einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der 65 Verbindungen der allgemeinen Formel (I) kommen vorzugsweise Salze von Metallen der II. bis IV. Hauptgruppe und der I. und II. sowie IV. bis VIII. Nebengruppe des Periodensystems in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol und Hinzufügen zur Verbindung der allgemeinen Formel I. Man kann Metallsalz-Komplexe in bekannter Weise, z.B. durch Abfiltrieren isolieren und gegebenenfalls durch Umkristallisieren reinigen.

Die vorliegende Erfindung betrifft weiterhin ein Mittel, zum Bekämpfen von unerwünschten Mikroorganismen, umfassend die erfindungsgemäßen Wirkstoffe. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man die erfindungsgemäßen Wirkstoffe auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln. Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die vorliegende Beschreibung betrifft weiterhin Saatgut, welches gemäß einem der im vorherigen Absatz beschriebenen Verfahren behandelt wurde. Die Saatgüter finden Anwendung in Verfahren zum Schutz von Saatgut vor unerwünschten Mikroorganismen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke mikrobizide Wirkung auf und können zur Bekämpfung von unerwünschten Mikroorganismen, wie Pilzen und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, *Brassica* Ölsaaten wie *Brassica napus* (z.B. Canola), *Brassica rapa, B. juncea* (z.B. (Acker-)Senf) und *Brassica carinata,* Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten, Kartoffeln, Pfeffer, Auberginen), *Liliaceae sp., Compositae sp.* (beispielsweise Salat, Artischocke and Chicoree - einschließlich Wurzelchicoree, Endivie oder gemeinen Chicoree), *Umbelliferae sp.* (beispielsweise Karrotte, Petersilie, Stangensellerie und Knollensellerie), *Cucurbitaceae sp.* (beispielsweise Gurke - einschließlich Gewürzgurke, Kürbis, Wassermelone, Flaschenkürbis und Melonen), *Alliaceae* sp. (beispielsweise Lauch und Zwiebel), *Cruciferae sp.* (beispielsweise Weißkohl, Rotkohl, Brokkoli, Blumenkohl, Rosenkohl, Pak Choi, Kohlrabi, Radieschen, Meerrettich, Kresse und Chinakohl), *Leguminosae sp.* (beispielsweise Erdnüsse, Erbsen, und Bohnen - wie z.B. Stangenbohne und Ackerbohne), *Chenopodiaceae sp.* (beispielsweise Mangold, Futterrübe, Spinat, Rote Rübe), *Malvaceae* (beispielsweise Okra), *Asparagaceae* (beispielsweise Spargel); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Beispiele für Nematoden-resistente Pflanzen sind z.B. folgenden US Patentanmeldungen beschrieben: 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 und 12/497,221.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-StressBedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im Allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d.h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. Pflanzen können mit verschiedenen Methoden tolerant gegenüber Glyphosate gemacht werden. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium* (Comai et al., 1983, Science 221, 370-371), das CP4-Gen des Bakteriums *Agrobacterium sp.* (Barry et al., 1992, Curr. Topics Plant Physiol. 7, 139-145), die Gene, die für eine EPSPS aus der Petunie (Shah et al., 1986, Science 233, 478-481), für eine EPSPS aus der Tomate (Gasser et al., 1988, J. Biol. Chem. 263, 4280-4289) oder für eine EPSPS aus Eleusine (WO 01/66704) kodieren. Es kann sich auch um eine mutierte EPSPS handeln. Glyphosate-tolerante Pflanzen können auch dadurch erhalten werden, dass man ein Gen exprimiert, das für ein Glyphosate-Oxidoreduktase-Enzym kodiert. Glyphosate-tolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert. Pflanzen, die EPSPS Gene, welche Glyphosate-Toleranz verleihen, exprimieren, sind beschrieben. Pflanzen, welche andere Gene, die Glyphosate-Toleranz verleihen, z.B. Decarboxylase-Gene, sind beschrieben.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym kodiert, transformiert werden, wie in WO 96/38567, WO 99/24585, WO 99/24586, WO 2009/144079, WO 2002/046387 oder US 6,768,044 beschrieben. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie in WO 2004/024928 beschrieben ist. Außerdem können Pflanzen noch toleranter gegen HPPD-Hemmern gemacht werden, indem man ein Gen in ihr Genom einfügt, welches für ein Enzym kodiert, das HPPD-Hemmer metabolisiert oder abbaut, wie z.B. CYP450 Enzyme (siehe WO 2007/103567 und WO 2008/150473).

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen wie z.B. in Tranel und Wright (Weed Science 2002, 50, 700-712) beschrieben ist. Die Herstellung von sulfonylharnstofftoleranten Pflanzen und imidazolinontoleranten Pflanzen ist beschrieben. Weitere sulfonylharnstoff- und imidazolinontolerante Pflanzen sind auch beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden (vgl. z.B. für Sojabohne US 5,084,082, für Reis WO 97/41218, für Zuckerrübe US 5,773,702 und WO 99/057965, für Salat US 5,198,599 oder für Sonnenblume WO 01/065922).

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, aufgelistet von Crickmore et al. (Microbiology and Molecular Biology Reviews 1998, 62, 807-813), aktualisiert von Crickmore et al. (2005) bei der *Bacillus thuringiensis* Toxin Nomenclatur, online bei: http://www.lifesci.sussex.ac.uk/Home/Neil_Crickmore/Bt/),
   oder insektizide Teile davon, z.B. Proteine der Cry-Proteinklassen Cry1Ab, Cry1Ac, Cry1B, Cry1C, Cry1D, Cry1F, Cry2Ab, Cry3Aa, or Cry3Bb oder insektizide Teile davon (z.B. EP-A 1999141 und WO 2007/107302), oder solche Proteine, kodiert durch synthetische Gene wie in US Patentanmeldung 12/249,016 beschrieben ist; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht (Nat. Biotechnol. 2001, 19, 668-72; Applied Environm. Microbiol. 2006, 71, 1765-1774) oder das binäre Toxin, das aus den Cry1A oder Cry1F Proteinen besteht und die Cry2Aa oder Cry2Ab oder Cry2Ae Proteine (US Patentanmeldung 12/214,022 und EP08010791.5); oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht (WO 94/21795); oder
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 5) bis 7) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Bautnwoll-Event COT 102; oder
9) ein sezerniertes Protein aus *Bacillus thuringiensis* oder *Bacillus cereus,* das in Gegenwart eines Kristallproteins von *Bacillus thuringiensis* insektizid wirkt, wie das binäre Toxin, das aus den Proteinen VIP3 und Cry1A oder Cry1F besteht (US Patentanmeldungen 61/126083 und 61/195019), oder das binäre Toxin, das aus dem VIP3 Protein und den Cry2Aa oder Cry2Ab oder Cry2Ae Proteinen besteht (US Patentanmeldung 12/214,022 und EP 08010791.5); oder
10) ein Protein gemäß Punkt 9) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt).

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 10 kodieren. In einer Ausführungsform enthält eine insektenresistente Pflanze mehr als ein Transgen, das für ein Protein nach einer der oben genannten 1 bis 10 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Eine "insekten-resistente transgene Pflanze" umfasst im vorliegenden Zusammenhang weiterhin jede Pflanze, die wenigstens ein Transgen enthält, welches eine Sequenz zur Herstellung einer Doppelstrang-RNA umfasst, die nach Nahrungsaufnahme durch einen Insektenschädling das Wachstum dieses Schädlings hindert.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.
4) Transgene Pflanzen oder Hybridpflanzen wie Zwiebeln mit bestimmten Eigenschaften wie "hohem Anteil an löslichen Feststoffen" (,high soluble solids content'), geringe Schärfe (,low pungency', LP) und/oder lange Lagerfähigkeit (,long storage', LS).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten, wie Baumwollpflanzen mit einer erhöhten Expression der Saccharosephosphatsynthase;
c) Pflanzen wie Baumwollpflanzen mit einer erhöhten Expression der Saccharosesynthase;
d) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
e) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Kartoffeln, welche Virus-resistent sind z.B. gegen den Kartoffelvirus Y (Event SY230 und SY233 von Tecnoplant, Argentinien), oder welche resistent gegen Krankheiten wie die Kraut- und Knollenfäule (potato late blight) (z.B. RB Gen), oder welche eine verminderte kälteinduzierte Süße zeigen (welche die Gene Nt-Inh, II-INV tragen) oder welche den Zwerg-Phänotyp zeigen (Gen A-20 Oxidase).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften im Samenausfall (seed shattering). Solche Pflanzen können durch genetische Transformation oder durch Selektion von Pflanzen, die eine Mutation enthalten, die solche veränderten Eigenschaften verleihen, und umfassen Pflanzen wie Raps mit verzögertem oder vermindertem Samenausfall.

Besonders nützliche transgene Pflanzen, die erfindungsgemäß behandelt werden können, sind Pflanzen mit Transformationsevents oder Kombinationen von Transformationsevent, welche in den USA beim Animal and Plant Health Inspection Service (APHIS) of the United States Department of Agriculture (USDA) Gegenstand von erteilten oder anhängigen Petitionen für den nicht-regulierten Status sind. Die Information hierzu ist jederzeit beim APHIS (4700 River Road Riverdale, MD 20737, USA) erhältlich, z.B. über die Internetseite http://www.aphis.usda.gov/brs/not_reg.html. Am Anmeldetag dieser Anmeldung waren beim APHIS die Petitionen mit folgenden Informationen entweder erteilt oder anhängig:
- Petition: Identifikationsnummer der Petition. Die Technische Beschreibung des Transformations-events kann im einzelnen Petitionsdokument erhältlich von APHIS auf der Website über die Petitionsnummer gefunden werden. Diese Beschreibungen sind hiermit per Referenz offenbart.
- Erweiterung einer Petition: Referenz zu einer frühere Petition, für die eine Erweiterung oder Verlängerung beantragt wird.
- Institution: Name der die Petition einreichenden Person.
- Regulierter Artikel: die betroffen Pflanzenspecies.
- Transgener Phänotyp: die Eigenschaft ("Trait"), die der Pflanze durch das Transformationsevent verliehen wird.
- Transformationevent oder -linie: der Name des oder der Events (manchmal auch als Linie(n) bezeichnet), für die der nicht-regulierte Status beantragt ist.
- APHIS Documente: verschiedene Dokumente, die von APHIS bzgl. der Petition veröffentlicht warden oder von APHIS auf Anfrage erhalten werden können.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://cera-gmc.org/index.php?evidcode=&hstIDXCode=&gType=&AbbrCode=&atCode=&stCode=&coID Code=&action=gm_crop_database&mode=Submit).

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen und Insekten, eingesetzt werden.

Weiter können die erfindungsgemäßen Verbindungen allein oder in Kombinationen mit anderen Wirkstoffen als Antifouling-Mittel eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator; Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola; Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).
Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Darüber hinaus weisen die erfindungsgemäßen Wirkstoffeauch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe können daher sowohl in medizinischen als auch in nicht-medizinischen Anwendungen eingesetzt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberellazeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die erfindungsgemäßen Wirkstoffe greifen in den Metabolismus der Pflanzen ein und können deshalb auch als Wachstumsregulatoren eingesetzt werden.

Pflanzenwachstumsregulatoren können verschiedenartige Wirkungen auf Pflanzen ausüben. Die Wirkungen der Stoffe hängen im Wesentlichen von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation ab. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträger bezogen auf die Bodenfläche erzielt werden können. Ein Vorteil der so erzielten kleineren Pflanzen ist auch, dass die Kultur leichter bearbeitet und geerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder größere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine große Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden ("Ausdünnung"), um die Alternanz zu brechen. Unter Alternanz versteht man die Eigenart einiger Obst-Arten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die aufgeführten Pflanzen können besonders vorteilhaft erfindungsgemäß mit den Verbindungen der allgemeinen Formel (I) bzw. den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

Die Erfindung wird durch die folgenden Beispiele veranschaulicht. Die Erfindung ist jedoch nicht auf die Beispiele limitiert.

### Herstellungsbeispiele

### Herstellung von Verbindung Nr. 3 (Verfahren A)

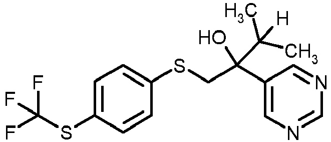

Zu 0.70 g (3.3 mmol) 4-[(Trifluormethyl)sulfanyl]benzolthiol gelöst in 20 ml N,N-Dimethylformamid wurden bei Raumtemperatur unter Argonatmosphäre 0.13 g (60%, 3.3 mmol) Natriumhydrid zugegeben und die Reaktionsmischung für 1 h bei Raumtemperatur gerührt. Anschließend wurden 0.5 g (3.0 mmol) 5-(2-Isopropyloxiran-2-yl)pyrimidin zugegeben und die Reaktionsmischung für 12 h bei 100 °C gerührt. Nach dem Abkühlen auf Raumtemperatur wurde das Lösungsmittel unter vermindertem Druck entfernt und der Rückstand mit gesättigter wässriger Natriumchloridlösung sowie Ethylacetat versetzt. Die organische Phase wurde abgetrennt, über Natriumsulfat getrocknet, filtriert und eingeengt. Das Rohprodukt wurde anschließend säulenchromatographisch (Cyclohexan/Essigsäureethylester 1:1) gereinigt. Man erhielt 0.11 g (10 %) des gewünschten Produktes.

### Herstellung von 5-(2-isopropyloxiran-2-yl)]pyrimidin

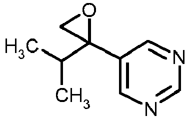

Unter einer Argonatmosphäre wurden zu 8.06 g (37 mmol) Trimethylsulfoxoniumiodid und 1.47 g Natriumhydrid (60%, 37 mmol) langsam 50 ml Dimethylsulfoxid zugetropft. Die Reaktionsmischung wurde anschließend für 15 min bei Raumtemperatur gerührt und es wurden 5.00 g (33 mmol) 2-Methyl-1-(5-pyrimidinyl)-1-propanon, gelöst in 10 ml Tetrahydrofuran zugegeben. Die Reaktionsmischung wurde für 90 min bei 50 °C gerührt. Anschließend wurde die Reaktionsmischung unter vermindertem Druck eingeengt und der Rückstand mit gesättigter wässriger Natriumchloridlösung sowie Ethylacetat versetzt. Die organische Phase wurde abgetrennt, über Natriumsulfat getrocknet, filtriert und eingeengt. Man erhielt 1.36 g (25%) des gewünschten Produktes, welches ohne weitere Aufreinigung umgesetzt wurde.

### Herstellung von Verbindung Nr. 12 (Verfahren B)

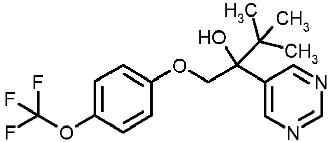

Ein Gemisch aus 3.8 g (13.6 mmol) 3,3-Dimethyl-1-[4-(trifluormethoxy)phenoxy]butan-2-on und 2.50g (15.6 mmol) 5-Brompyrimidin in einer Mischung aus 20 ml trockenem Tetrahydrofuran und 20 ml trockenem Diethylether wurde unter Argonatmosphäre auf -120 °C gekühlt. Unter Rühren wurde anschließend *n*-Butyllithium (6.55 ml, 2.5M, 16.3 mmol) langsam zugegeben. Nach vollendeter Zugabe wurde die Reaktionsmischung über Nacht langsam auf Raumtemperatur erwärmt. Die Reaktionsmischung wurde mit 100 ml einer 10%igen wässrigen Ammoniumchlorid-Lösung versetzt und die organische Phase abgetrennt. Die organische Phase wurde anschließend mit 1N Salzsäure und gesättigter wässriger Natriumchlorid-Lösung gewaschen, über Natriumsulfat getrocknet, filtriert und eingeengt. Das Rohprodukt wurde anschließend säulenchromatographisch (Cyclohexan/Essigsäureethylester 1:1) gereinigt. Man erhielt 1.46 g (30 %) des gewünschten Produktes.

### Herstellung von Verbindung Nr. 9 (Verfahren C)

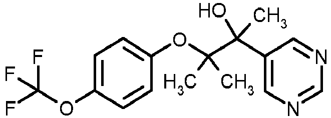

Zu 0.33 g (1.0 mmol) 2-Methyl-1-(pyrimidin-5-yl)-2-[4-(trifluormethoxy)phenoxy]propan-1-on gelöst in 10 ml Toluol wurden bei -78 °C unter Argonatmosphäre 1.0 ml (3.0 M Lösung in Diethylether, 3.0 mmol) Methylmagnesiumbromid zugegeben und die Reaktionsmischung für 30 min bei -78 °C gerührt. Die Reaktionsmischung wurde mit 100 ml einer gesättigen wässrigen Ammoniumchlorid-Lösung und mit 100 ml Toluol verrührt und die organische Phase abgetrennt. Die organische Phase wurde über Natriumsulfat getrocknet, filtriert und eingeengt. Das Rohprodukt wurde anschließend säulenchromatographisch gereinigt. Man erhielt 0.12 g (36 %) des gewünschten Produktes.

In analoger Form erhält man die Verbindungen 9-11, 13-16, 22-27.

### Herstellung von 2-Methyl-1-(pyrimidin-5-yl)-2-[4-(trifluormethoxy)phenoxy]propan-1-on

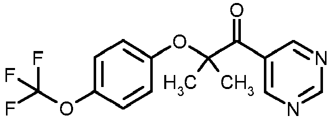

Ein Gemisch aus 4.4 g (25.0 mmol) 4-Trifluormethoxyphenol und 15.5 g (112.5 mmol) Kaliumcarbonat in 37.5 ml trockenem Dimethylsulfoxid wurde 2 h bei Raumtemperatur gerührt. Die Reaktionsmischung wurde anschließend auf 60 °C erwärmt, und es wurden 25.7 g (112.5 mmol) 2-Brom-2-methyl-1-(pyrimidin-5-yl)propan-1-on, gelöst in 12.5 ml Dimethylsulfoxid zugegeben. Nach vollendeter Zugabe wurde die Reaktionsmischung über Nacht gerührt. Die Reaktionsmischung wurde auf 250 ml Wasser gegossen, und mit 250 ml Essigester ausgeschüttelt. Die organische Phase wurde abgetrennt, und anschließend mit gesättigter wässriger Natriumchlorid-Lösung gewaschen, über Natriumsulfat getrocknet, filtriert und eingeengt. Das Rohprodukt wurde anschließend säulenchromatographisch gereinigt. Man erhielt 1.24 g (14 %) des gewünschten Produktes.

### Herstellung von 2-Brom-2-methyl-1-(pyrimidin-5-yl)propan-1-on

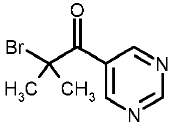

Zu 28.7 g (191.5 mmol) 2-Methyl-1-(pyrimidin-5-yl)propan-1-on gelöst in 463 ml Bromwasserstoffsäure wurden bei Raumtemperatur 11.8 mL (229.8 mmol) Brom, gelöst in 111.5 ml Bromwasserstoffsäure zugegeben. Nach vollendeter Zugabe wurde die Reaktionsmischung über Nacht gerührt. Anschließend wurde die Reaktionsmischung unter vermindertem Druck eingeengt. Man erhielt 50.8 g (91%) des gewünschten Produktes, welches ohne weitere Aufreinigung umgesetzt wurde.

### Herstellung von 3,3-Dimethyl-1-[(trifluormethoxy)phenoxy]butan-2-on (Verfahren E)

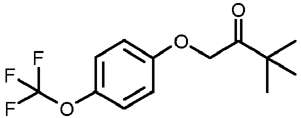

Zu einer Mischung aus 5.5 g (31 mmol) 4-Trifluormethoxyphenol und 4.27g (31 mmol) Kaliumcarbonat in 100 ml Acetonitril wurde unter Rühren bei Raumtemperatur eine Lösung von 5.5 g (31 mmol) 1-Brom-3,3-dimethylbutan-2-on in 50 ml Acetonitril langsam zugetropft. Nach vollendeter Zugabe wurde die Reaktionsmischung über Nacht bei Rückflusstemperatur gerührt. Der Feststoff wurde abfiltriert und das Filtrat eingeengt. Der Rückstand wurde in 50 ml Essigester aufgenommen und die organische Phase mit eiskalter 1M wässriger Natriumhydroxid-Lösung sowie Wasser gewaschen. Die organische Phase wurde abgetrennt, über Natriumsulfat getrocknet, filtriert und eingeengt. Das Rohprodukt wurde anschließend säulenchromatographisch gereinigt. Man erhielt 2.22 g (26 %) des gewünschten Produktes.

**Tabelle 1**

| | | | | | | | | | | |
|---|---|---|---|---|---|---|---|---|---|---|
| 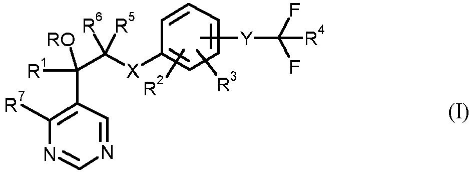 | | | | | | | | | | |

| **Nr.** | **X** | **R** | **R¹** | **R²** | **R³** | -**YCF₂R⁴** | **R⁵** | **R⁶** | **R⁷** | **Physikalische Daten** |
|---|---|---|---|---|---|---|---|---|---|---|
| 1* | O | H | Me | H | H | 4-OCF₃ | H | H | H | |
| 2* | O | H | iPr | H | H | 4-OCF₃ | H | H | H | |
| 3* | S | H | iPr | H | H | 4-SCF₃ | H | H | H | LogP 3,28^{[a]}, [M+H]⁺=375 |
| 4 | S | H | tBu | H | H | 4-SCF₃ | H | H | H | ¹H-NMR (400 MHz, DMSO-d₆): δ = 0.93 (s, 9H), 3.56 (d, J = 12 Hz, 1H), 4.12 (d, J = 12 Hz, 1H), 5.56 (s, 1H), 7.44 (d, J = 8 Hz, 2H), 7.58 (d, J = 8 Hz, 2H), 8.82 (s, 2H), 9.04 (s, 1H) ppm. |
| 5* | O | H | Me | H | H | 4-SCF₃ | Me | Me | H | LogP 2,56 ^{[a]}, [M+H]⁺=358 |
| 6 | O | H | 2,4-Difluorphenyl | H | H | 4-OCF₃ | -CH₂-CH₂- | | H | ¹H-NMR (400 MHz, DMSO-d₆): δ = 0.90-0.99 (m, 1H), 1.04-1.10 (m, 1H), 1.32-1.45 (m, 2H), 6.47-6.51 (m, 3H), 6.92-7.02 (m, 3H), 7.22 (td, 1H), 8.09 (dd, 1H), 8.77 (s, 2H); 9.06 (s, 1H) ppm. |
| 7 | CH₂ | H | tBu | H | H | 4-SCF₃ | H | H | H | ¹H-NMR (400 MHz, DMSO-d₆): δ = 0.89 (s, 9H), 2.09-2.21 (m, 2H), 2.52 (ddd, 1H), 2.65 (ddd, 1H), 3.32 (bs, 1H), 7.32 (d, 2H), 7.60 (d, 2H), 8.83 (s, 2H), 9.03 (s, 1H) ppm. |
| 8 | O | H | 2,4-Difluorphenyl | H | H | 4-SCF₃ | -CH₂-CH₂- | | H | ¹H-NMR (400 MHz, DMSO-d₆): δ = 1.01-1.16 (m, 2H), 1.36-1.49 (m, 2H), 6.55-6.59 (m, 3H), 6.88 (td, 1H), 7.22 (td, 1H), 7.32 (d, 2H), 8.08 (dd, 1H), 8.77 (s, 2H); 9.04 (s, 1H) ppm. |
| 9* | O | H | Me | H | H | 4-OCF₃ | Me | Me | H | ¹H-NMR (400 MHz, DMSO-d₆): δ = 1.01 (s, 3H), 1.33 (s, 3H), 1.70 (s, 3H), 5.76 (s, 1H), 6.99 (dd, 2H), 7.25 (dd, 2H), 8.94 (s, 2H); 9.08 (s, 1H) ppm. |
| 10* | S | H | Me | H | H | 4-OCF₃ | Me | Me | H | ¹H-NMR (400 MHz, DMSO-d₆): δ = 1.11 (s, 3H), 1.16 (s, 3H), 1.73 (s, 3H), 5.74 (s, 1H), 7.31 (dd, 2H), 7.54 (dd, 2H), 8.95 (s, 2H); 9.07 (s, 1H) ppm. |
| 11* | S | H | Me | H | H | 4-SCF₃ | Me | Me | H | ¹H-NMR (400 MHz, DMSO-d₆): δ = 1.11 (s, 3H), 1.16 (s, 3H), 1.73 (s, 3H), 5.78 (s, 1H), 7.58 (dd, 2H), 7.65 (dd, 2H), 8.95 (s, 2H); 9.07 (s, 1H) ppm. |
| 12 | O | H | tBu | H | H | 4-OCF₃ | H | H | H | ¹H-NMR (400 MHz, DMSO-d₆): δ = 0.92 (s, 9H), 4.23 (d, J = 10 Hz, 1H), 4.85 (d, J = 10 Hz, 1H), 5.53 (s, 1H), 7.00 (d, J = 8 Hz, 2H), 7.35 (d, J = 8 Hz, 2H), 8.83 (s, 2H), 9.04 (s, 1H) ppm. |
| 13 | S | H | 1-Propinyl | H | H | 4-OCF₃ | Me | Me | H | ¹H-NMR (400 MHz, DMSO-d₆): δ = 1.23 (s, 3H), 1.24 (s, 3H), 1.90 (s, 3H), 6.71 (s, 1H), 7.31 (dd, 2H), 7.55 (dd, 2H), 9.00 (s, 2H); 9.13 (s, 1H) ppm. |
| 14 | O | H | 1-Propinyl | H | H | 4-OCF₃ | Me | Me | H | ¹H-NMR (400 MHz, DMSO-d₆): δ = 1.14 (s, 3H), 1.38 (s, 3H), 1.97 (s, 3H), 6.74 (s, 1H), 6.98 (dd, 2H), 7.25 (dd, 2H), 8.96 (s, 2H); 9.13 (s, 1H) ppm. |
| 15 | S | H | 1-Propinyl | H | H | 4-SCF₃ | Me | Me | H | ¹H-NMR (400 MHz, DMSO-d₆): δ = 1.26 (s, 3H), 1.27 (s, 3H), 1.88 (s, 3H), 6.75 (s, 1H), 7.60 (dd, 2H), 7.65 (dd, 2H), 9.90 (s, 2H); 9.12 (s, 1H) ppm. |
| 16* | S | H | 2-Propenyl | H | H | 4-SCF₃ | Me | Me | H | ¹H-NMR (400 MHz, DMSO-d₆): δ = 1.15 (s, 3H), 1.28 (s, 3H), 2.80-2.88 (m, 1H), 3.10-3.22 (m, 1H), 4.96 (d, 1H), 5.11 (d, 1H), 5.50-5.62 (m, 1H), 5.70-5.85 (m, 1H), 7.57 (dd, 2H), 7.68 (dd, 2H), 8.86 (s, 2H); 9.02 (s, 1H) ppm. |
| 17 | O | H | tBu | H | H | 4-SCF₃ | H | H | H | ¹H-NMR (400 MHz, DMSO-d₆): δ = 0.91 (s, 9H), 4.28 (d, J = 10 Hz, 1H), 4.91 (d, J = 10 Hz, 1H), 5.61 (s, 1H), 7.07 (d, J = 7 Hz, 2H), 7.60 (d, J = 7 Hz, 2H), 8.84 (s, 2H), 9.05 (s, 1H) ppm. |
| 18 | O | H | MCP | H | H | 4-OCF₃ | H | H | H | ¹H-NMR (400 MHz, DMSO-d₆): δ = 0.11 (m, 2H), 0.32 (m, 2H), 0.95 (s, 3H), 4.16 (d, J = 10 Hz, 1H), 4.74 (d, J = 10 Hz, 1H), 5.62 (s, 1H), 7.06 (d, J = 9 Hz, 2H), 7.28 (d, J = 9 Hz, 2H), 8.89 (s, 2H), 9.09 (s, 1H) ppm. |
| 19 | O | H | MCP | H | H | 4-SCF₃ | H | H | H | LogP 3,30 [a], [M+H]⁺=371 |
| 20 | O | H | CCP | H | H | 4-OCF₃ | H | H | H | ¹H-NMR (400 MHz, DMSO-d₆): δ = 0.89 (m, 1H), 1.12 (m, 1H), 1.45 (m, 1H), 1.57 (m, 1H), 4.44 (d, J = 10 Hz, 1H), 4.74 (d, J = 10 Hz, 1H), 6.31 (s, 1H), 7.10 (d, J = 7 Hz, 2H), 7.29 (d, J = 7 Hz, 2H), 8.98 (s, 2H), 9.17 (s, 1H) ppm. |
| 21 | O | H | CCP | H | H | 4-SCF₃ | H | H | H | ¹H-NMR (400 MHz, DMSO-d₆): δ = 0.88 (m, 1H), 1.11 (m, 1H), 1.47 (m, 1H), 1.57 (m, 1H), 4.50 (d, J = 10 Hz, 1H), 4.80 (d, J = 10 Hz, 1H), 6.34 (s, 1H), 7.15 (d, J = 9 Hz, 2H), 7.64 (d, J = 9 Hz, 2H), 8.99 (s, 2H), 9.15 (s, 1H) ppm. |
| 22* | O | H | Me | H | H | 3-OCF₃ | Me | Me | H | ¹H-NMR (400 MHz, DMSO-d₆): δ = 1.04 (s, 3H), 1.34 (s, 3H), 1.70 (s, 3H), 5.76 (s, 1H), 6.87 (br s, 1H), 6.95 (br d, 1H), 7.05 (br d, 1H), 7.38 (t, 1H), 8.94 (s, 2H); 9.07 (s, 1H) ppm. |
| 23 | O | H | 1-Propinyl | H | H | 3-OCF₃ | Me | Me | H | ¹H-NMR (400 MHz, DMSO-d₆): δ = 1.04 (s, 3H), 1.34 (s, 3H), 1.70 (s, 3H), 5.76 (s, 1H), 6.87 (br s, 1H), 6.95 (br d, 1H), 7.05 (br d, 1H), 7.38 (t, 1H), 8.94 (s, 2H); 9.07 (s, 1H) ppm. |
| 24* | O | H | iPr | H | H | 3-OCF₃ | Me | Me | H | ¹H-NMR (400 MHz, DMSO-d₆): δ = 0.91 (d, 3H), 0.97 (d, 3H), 1.09 (s, 3H), 1.11-1.21 (m, 1H), 1.31 (s, 3H), 6.98 (br s, 1H), 7.05 (br d, 1H), 7.09 (br d, 1H), 7.42 (t, 1H), 9.01 (s, 2H); 9.07 (s, 1H) ppm. |
| 25* | O | H | Me | H | H | 2-OCF₃ | Me | Me | H | ¹H-NMR (400 MHz, DMSO-d₆): δ = 1.10 (s, 3H), 1.31 (s, 3H), 1.72 (s, 3H), 5.78 (s, 1H), 7.14-7.21 (m, 2H), 7.27-7.31 (m, 1H), 7.35 (d, 1H), 8.88 (s, 2H); 9.05 (s, 1H) ppm. |
| 26 | O | H | 1-Propinyl | H | H | 2-OCF₃ | Me | Me | H | ¹H-NMR (400 MHz, DMSO-d₆): δ = 1.10 (s, 3H), 1.40 (s, 3H), 1.58 (s, 3H), 7.01-7.39 (m, 4H), 8.95 (s, 2H), 9.11 (s, 1H) ppm. |
| 27* | O | H | iPr | H | H | 2-OCF₃ | Me | Me | H | ¹H-NMR (400 MHz, DMSO-d₆): δ = 0.91 (d, 3H), 0.98 (d, 3H), 1.08 (s, 3H), 1.33 (s, 3H), 2.63-2.70 (m, 1H), 7.18-7.40 (m, 4H), 8.99 (s, 2H); 9.07 (s, 1H) ppm. |
| 28 | O | H | tBu | H | H | 4-OCF₂Cl | H | H | H | LogP 3,40 ^{[a]}, [M+H]⁺=373 |
| 29* | O | H | tBu | 2-Cl | H | 4-OCF₃ | H | H | H | LogP 3,47 ^{[a]}, [M+H]⁺=391 |
| 30* | O | H | tBu | 2-Me | H | 4-OCF₃ | H | H | H | LogP 3,59 ^{[a]}, [M+H]⁺=371 |
| 31* | O | H | tBu | 3-Cl | H | 4-SCF₃ | H | H | H | LogP 3,76 ^{[a]}, [M+H]⁺=407 |
| 32* | O | H | tBu | 3-Me | H | 4-SCF₃ | H | H | H | LogP 3,04 ^{[a]}, [M+H]⁺=387 |
| 33 | O | H | tBu | H | H | 4-SO₂CF₃ | H | H | H | LogP 2,82 ^{[a]}, [M+H]⁺=405 |
| 34 | O | H | tBu | H | H | 4-OCF₂H | H | H | H | LogP 2,63 ^{[a]}, [M+H]⁺=339 |

| | | | | | | | | | | |
|---|---|---|---|---|---|---|---|---|---|---|
| * zum Vergleich Me = Methyl, iPr = Isopropyl, tBu = tert-Butyl, CCP = 1-Chlorcyclopropyl, MCP = 1-Methylcyclopropyl | | | | | | | | | | |

Die Messung der logP Welle erfolgte gemäß EEC Directive 79/831 Annex V.A8 durch HPLC (High Performance Liquid Chromatography) an reversed-phase Säulen (C 18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten linearer Gradient von 10% Acetonitril bis 95% Acetonitril

### Anwendungsbeispiele

### Beispiel A: Sphaerotheca-Test (Gurke) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von *Sphaerotheca fuliginea* inokuliert. Anschließend werden die Pflanzen in einem Gewächshaus bei 70 % relativer Luftfeuchtigkeit und einer Temperatur von 23°C aufgestellt. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 500 ppm einen Wirkungsgrad von 70 % oder mehr:

**Tabelle A: Sphaerotheca-Test (Gurke) / protektiv**

| Nr. | Wirkstoff | Aufwandmenge (ppm) | Wirkungsgrad (%) |
|---|---|---|---|
| 2* | 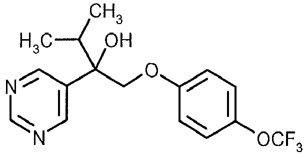 | 500 | 99 |
| 3* | 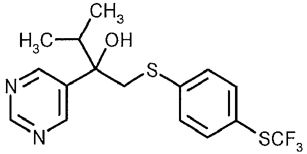 | 500 | 94 |
| 4 | 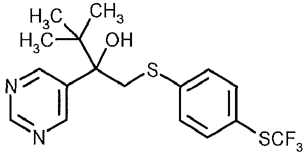 | 500 | 91 |
| 7 | 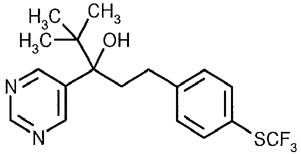 | 500 | 95 |
| 8 | 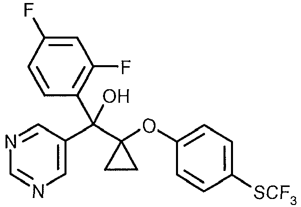 | 500 | 100 |
| 9* | 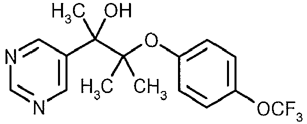 | 500 | 95 |
| 22* | 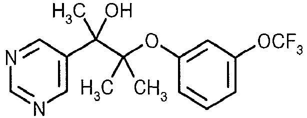 | 500 | 95 |
| 12 | 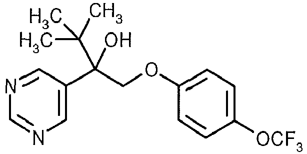 | 500 | 100 |
| 13 | 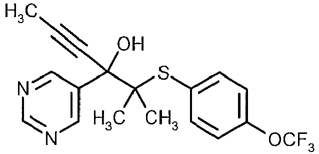 | 500 | 76 |
| 14 | 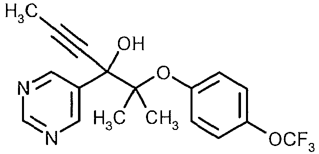 | 500 | 95 |
| 23 | 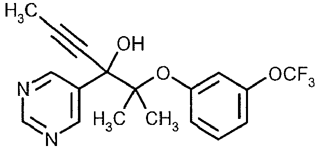 | 500 | 88 |
| 15 | 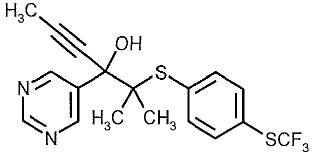 | 500 | 88 |
| 24* | 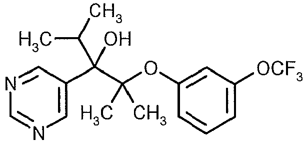 | 500 | 83 |
| 27* | 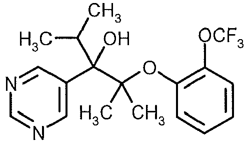 | 500 | 88 |
| 17 | 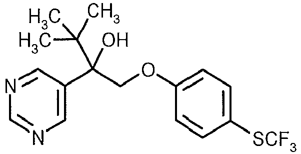 | 500 | 95 |
| 18 | 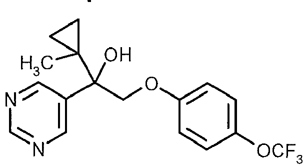 | 500 | 95 |
| 19 | 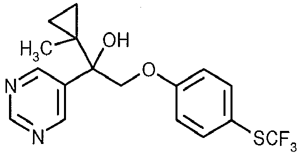 | 500 | 95 |
| 20 | 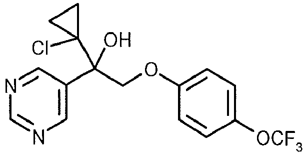 | 500 | 75 |
| 21 | 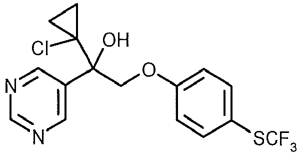 | 500 | 100 |
| 29* | 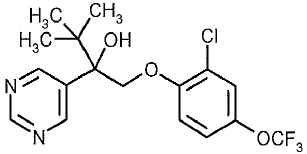 | 500 | 100 |
| 30* | 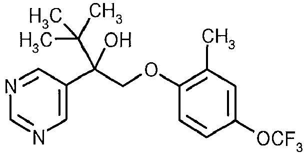 | 500 | 100 |
| 31* | 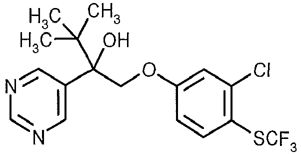 | 500 | 100 |
| 32* | 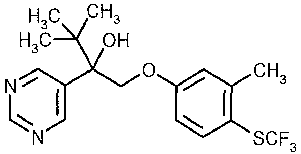 | 500 | 95 |
| 33 | 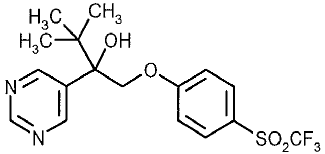 | 500 | 95 |
| 34 | 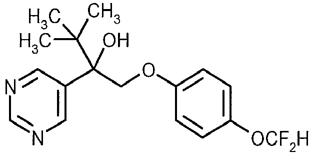 | 500 | 95 |

| | | | |
|---|---|---|---|
| * zum Vergleich | | | |

### Beispiel B: Alternaria-Test (Tomate) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24 h bei 100 % relativer Feuchte und 22°C. Anschließend stehen die Pflanzen bei 96 % relativer Luftfeuchtigkeit und einer Temperatur von 20°C. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 500 ppm einen Wirkungsgrad von 70 % oder mehr:

**Tabelle B: Alternaria-Test (Tomate) / protektiv**

| Nr. | Wirkstoff | Aufwandmenge (ppm) | Wirkungsgrad (%) |
|---|---|---|---|
| 2* | 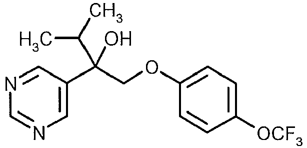 | 500 | 100 |
| 3* | 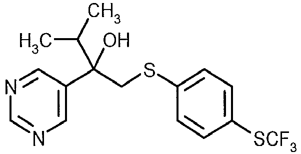 | 500 | 95 |
| 4 | 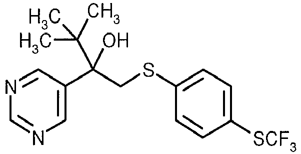 | 500 | 95 |
| 7 | 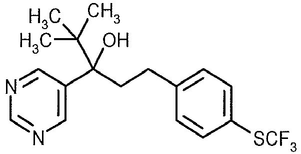 | 500 | 94 |
| 8 | 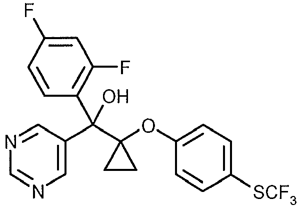 | 500 | 80 |
| 9* | 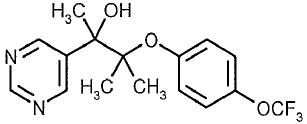 | 500 | 100 |
| 22* | 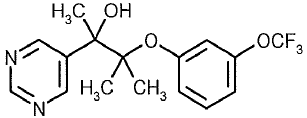 | 500 | 100 |
| 25* | 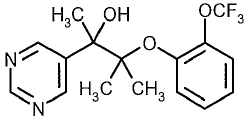 | 500 | 90 |
| 10* | 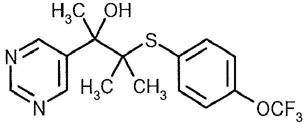 | 500 | 100 |
| 11* | 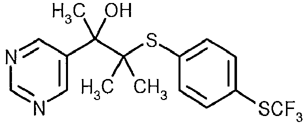 | 500 | 100 |
| 12 | 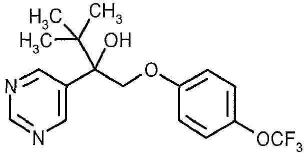 | 500 | 100 |
| 13 | 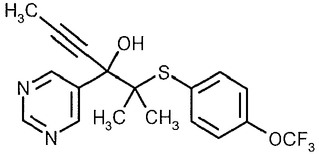 | 500 | 100 |
| 14 | 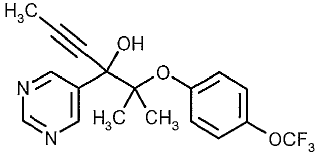 | 500 | 94 |
| 23 | 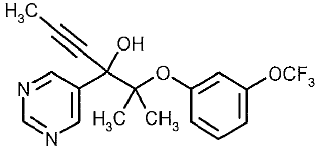 | 500 | 75 |
| 15 | 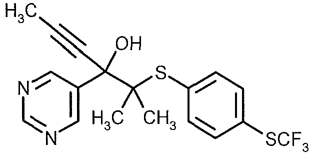 | 500 | 94 |
| 24* | 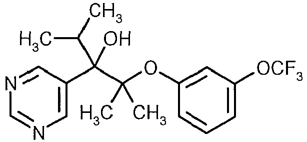 | 500 | 95 |
| 16* | 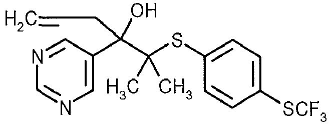 | 500 | 100 |
| 17 | 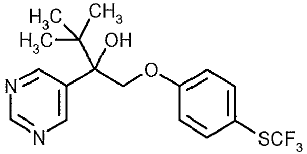 | 500 | 94 |
| 18 | 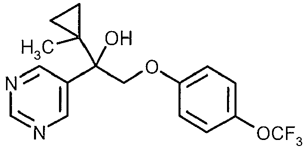 | 500 | 90 |
| 19 | 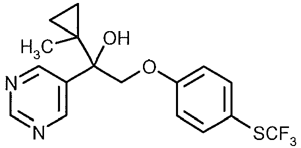 | 500 | 90 |
| 20 | 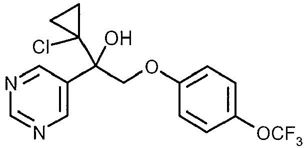 | 500 | 100 |
| 21 | 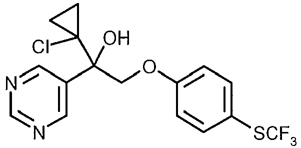 | 500 | 95 |
| 29* | 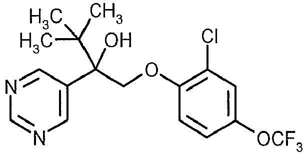 | 500 | 78 |
| 30* | 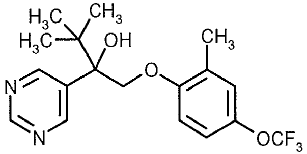 | 500 | 90 |
| 31* | 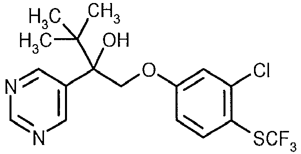 | 500 | 95 |
| 33 | 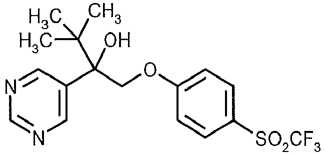 | 500 | 89 |
| 34 | 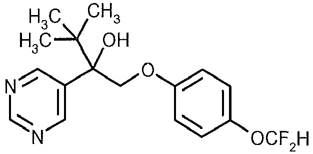 | 500 | 95 |

| | | | |
|---|---|---|---|
| * zum Vergleich | | | |

### Beispiel C: Leptosphaeria nodorum-Test (Weizen) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit bespritzt man junge Weizenpflanzen mit der Wirkstoffzubereitung in der angegebenen Aufwandmenge. 1 Tag nach der Behandlung werden die Pflanzen mit einer wässrigen Sporensuspension von *Leptosphaeria nodorum* inokuliert und verbleiben dann 48 h bei 100 % relativer Luftfeuchte und 22°C. Anschließend werden die Pflanzen in einem Gewächshaus bei 90 % relatviver Luftfeuchtigkeit und einer Temperatur von 22°C aufgestellt. 7-9 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 500 ppm einen Wirkungsgrad von 70 % oder mehr:

**Tabelle C: Leptosphaeria nodorum-Test (Weizen) / protektiv**

| Nr. | Wirkstoff | Aufwandmenge (ppm) | Wirkungsgrad (%) |
|---|---|---|---|
| 2* | 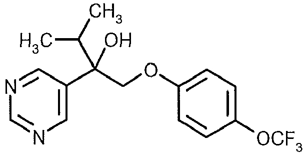 | 500 | 100 |
| 3* | 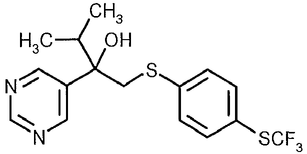 | 500 | 90 |
| 4 | 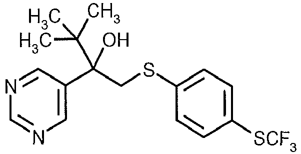 | 500 | 70 |
| 7 | 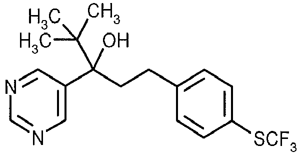 | 500 | 95 |
| 9* | 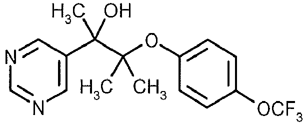 | 500 | 100 |
| 22* | 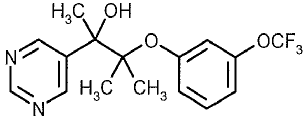 | 500 | 100 |
| 10* | 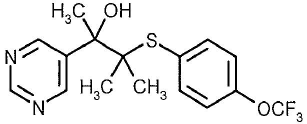 | 500 | 100 |
| 11* | 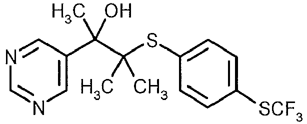 | 500 | 95 |
| 12 | 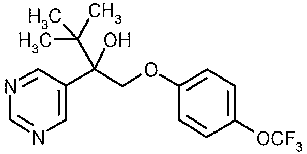 | 500 | 95 |
| 13 | 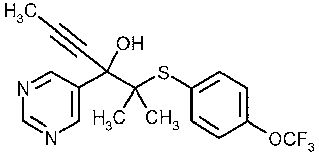 | 500 | 95 |
| 14 | 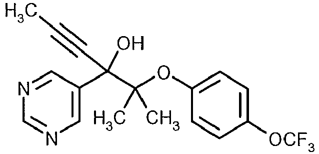 | 500 | 100 |
| 23 | 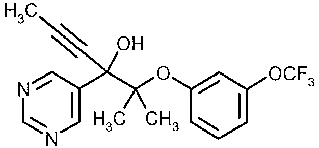 | 500 | 70 |
| 15 | 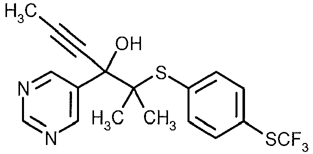 | 500 | 95 |
| 24 | 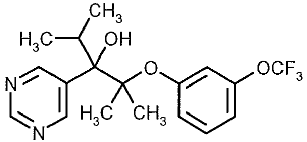 | 500 | 80 |
| 16* | 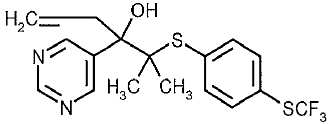 | 500 | 70 |
| 17 | 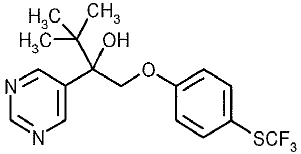 | 500 | 94 |
| 18 | 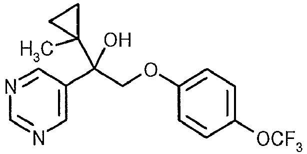 | 500 | 95 |
| 19 | 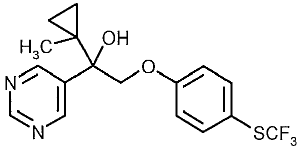 | 500 | 95 |
| 20 | 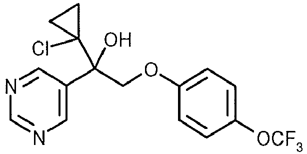 | 500 | 95 |
| 29* | 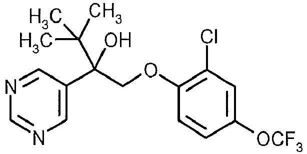 | 500 | 75 |
| 30* | 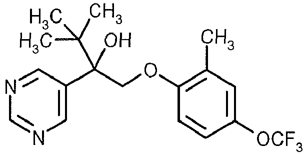 | 500 | 89 |
| 31* | 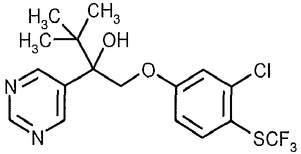 | 500 | 95 |
| 33 | 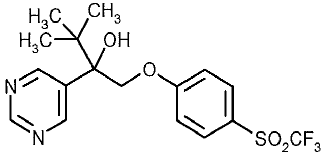 | 500 | 94 |
| 34 | 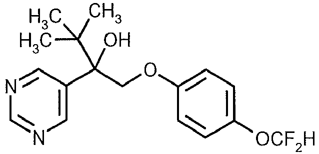 | 500 | 100 |

| | | | |
|---|---|---|---|
| * zum Vergleich | | | |

### Beispiel D: Venturia-Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine. Die Pflanzen werden dann im Gewächshaus bei ca. 21°C und einer relativen Luftfeuchtigkeit von ca. 90 % aufgestellt. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 100 ppm einen Wirkungsgrad von 70 % oder mehr:

**Tabelle D: Venturia-Test (Apfel) / protektiv**

| Nr. | Wirkstoff | Aufwandmenge (ppm) | Wirkungsgrad (%) |
|---|---|---|---|
| 2* | 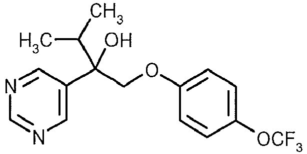 | 100 | 94 |
| 22* | 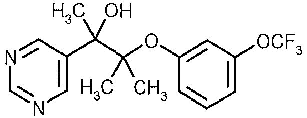 | 100 | 94 |
| 9* | 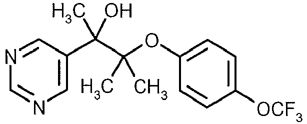 | 100 | 94 |
| 12 | 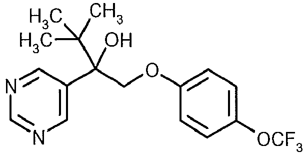 | 100 | 99 |
| 17 | 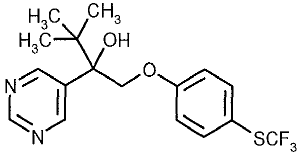 | 100 | 100 |
| 18 | 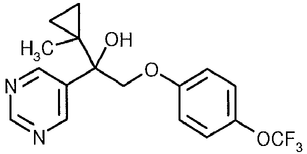 | 100 | 99 |
| 20 | 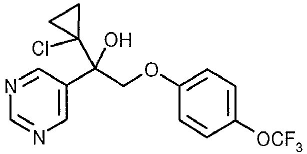 | 100 | 100 |
| 30* | 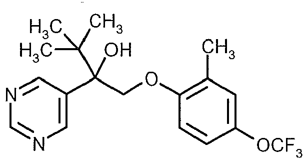 | 100 | 98 |
| 31* | 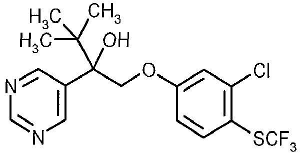 | 100 | 99 |
| 33 | 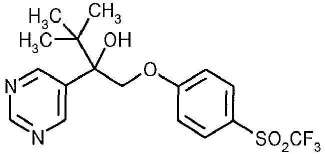 | 100 | 99 |

| | | | |
|---|---|---|---|
| * zum Vergleich | | | |

### Beispiel E: Uromyces-Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Bohnenrosterregers *Uromyces appendiculatus* inokuliert und verbleiben dann 1 Tag bei ca. 20°C und 100 % relativer Luftfeuchtigkeit in einer Inkubationskabine. Die Pflanzen werden dann im Gewächshaus bei ca. 21°C und einer relativen Luftfeuchtigkeit von ca. 90 % aufgestellt. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 100 ppm einen Wirkungsgrad von 70 % oder mehr:

**Tabelle E: Uromyces-Test (Bohne) / protektiv**

| Nr. | Wirkstoff | Aufwandmenge (ppm) | Wirkungsgrad (%) |
|---|---|---|---|
| 2* | 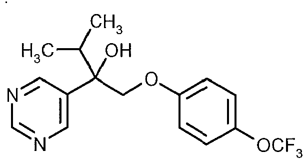 | 100 | 100 |
| 9* | 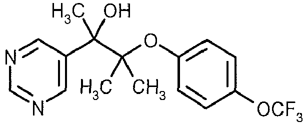 | 100 | 100 |
| 12 | 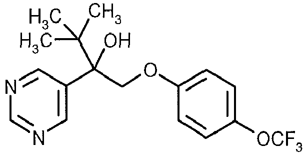 | 100 | 100 |
| 17 | 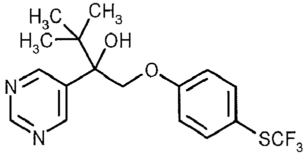 | 100 | 100 |
| 18 | 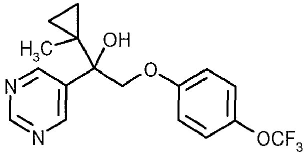 | 100 | 100 |
| 20 | 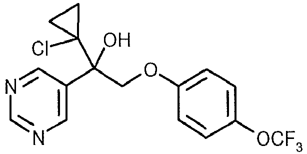 | 100 | 98 |
| 30* | 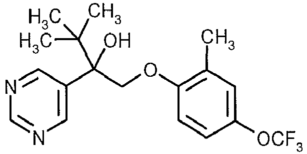 | 100 | 100 |
| 31* | 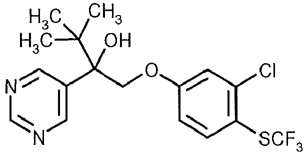 | 100 | 100 |
| 33 | 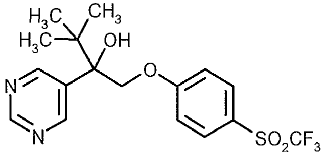 | 100 | 100 |

| | | | |
|---|---|---|---|
| * zum Vergleich | | | |

### Beispiel F: Blumeria graminis-Test (Gerste) / protektiv

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von *Blumeria graminis f.sp. hordei* bestäubt. Die Pflanzen werden in einem Gewächshaus bei einer Temperatur von ca. 18°C und einer relativen Luftfeuchtigkeit von ca. 80 % aufgestellt, um die Entwicklung von Mehltaupusteln zu begünstigen. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 500 ppm einen Wirkungsgrad von 70 % oder mehr:

**Tabelle F: Blumeria graminis-Test (Gerste) / protektiv**

| Nr. | Wirkstoff | Aufwandmenge (ppm) | Wirkungsgrad (%) |
|---|---|---|---|
| 2* | 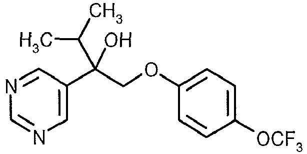 | 500 | 100 |
| 3* | 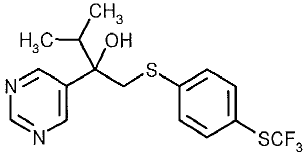 | 500 | 100 |
| 4 | 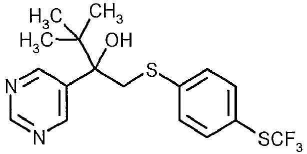 | 500 | 89 |
| 9* | 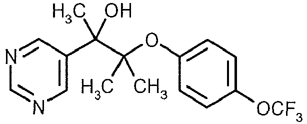 | 500 | 100 |
| 12 | 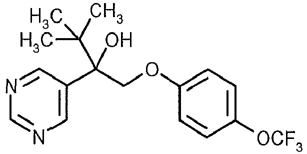 | 500 | 100 |
| 14 | 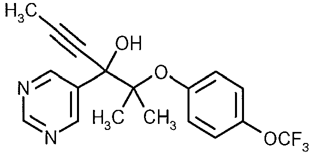 | 500 | 100 |
| 17 | 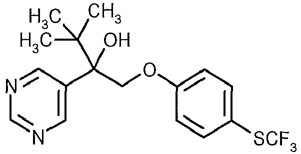 | 500 | 100 |
| 18 | 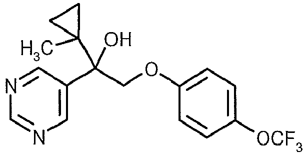 | 500 | 100 |
| 19 | 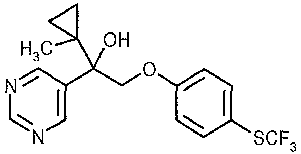 | 500 | 100 |
| 30* | 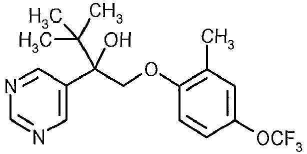 | 500 | 100 |
| 33 | 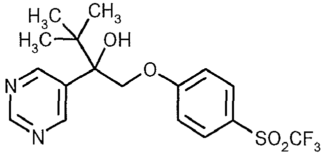 | 500 | 100 |

| | | | |
|---|---|---|---|
| * zum Vergleich | | | |

### Beispiel G: Septoria tritici-Test (Weizen) / protektiv

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von *Septoria tritici* besprüht. Die Pflanzen verbleiben 48 Stunden bei 20°C und 100 % relativer Luftfeuchtigkeit in einer Inkubationskabine. Danach werden die Pflanzen für weitere 60 Stunden unter eine Klarsichthaube bei 15°C und 100 % relativer Luftfeuchte gestellt. Die Pflanzen werden in einem Gewächshaus bei einer Temperatur von ca. 15°C und einer relativen Luftfeuchtigkeit von 80 % aufgestellt. 21 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 500 ppm einen Wirkungsgrad von 70 % oder mehr:

**Tabelle G: Septoria tritici-Test (Weizen) / protektiv**

| Nr. | Wirkstoff | Aufwandmenge (ppm) | Wirkungsgrad (%) |
|---|---|---|---|
| 2* | 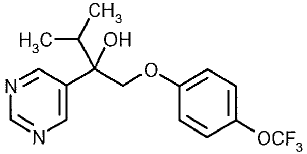 | 500 | 93 |
| 3* | 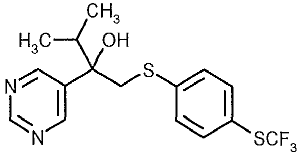 | 500 | 75 |
| 4 | 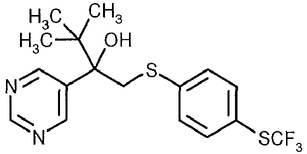 | 500 | 100 |
| 9* | 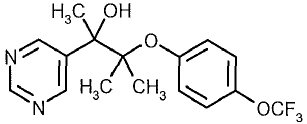 | 500 | 90 |
| 12 | 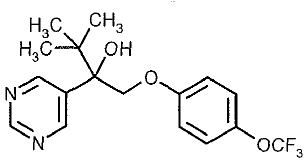 | 500 | 100 |
| 14 | 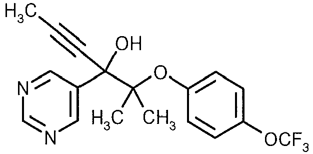 | 500 | 90 |
| 17 | 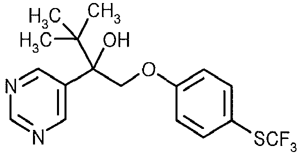 | 500 | 100 |
| 18 | 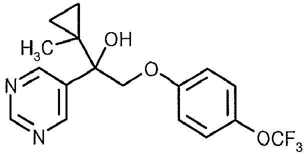 | 500 | 100 |
| 19 | 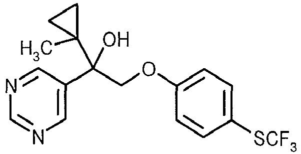 | 500 | 86 |
| 30* | 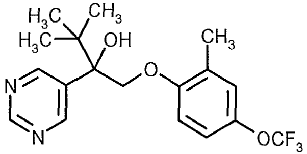 | 500 | 71 |

| | | | |
|---|---|---|---|
| * zum Vergleich | | | |

### Beispiel H: Puccinia triticina-Test (Weizen) / protektiv

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen mit einer Sporensuspension von *Puccinia triticina* besprüht. Die Pflanzen verbleiben 48 Stunden bei 20°C und 100 % relativer Luftfeuchtigkeit in einer Inkubationskabine. Die Pflanzen werden in einem Gewächshaus bei einer Temperatur von ca. 20°C und einer relativen Luftfeuchtigkeit von ca. 80 % aufgestellt. 8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 500 ppm einen Wirkungsgrad von 70 % oder mehr:

**Tabelle H: Puccinia triticina-Test (Weizen) / protektiv**

| Nr. | Wirkstoff | Aufwandmenge (ppm) | Wirkungsgrad (%) |
|---|---|---|---|
| 2* | 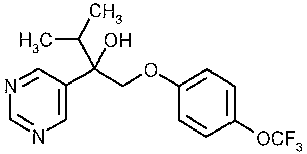 | 500 | 100 |
| 9* | 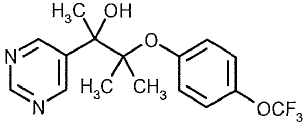 | 500 | 100 |
| 12 | 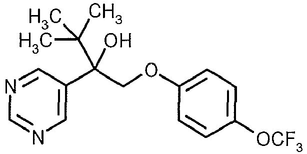 | 500 | 100 |
| 14 | 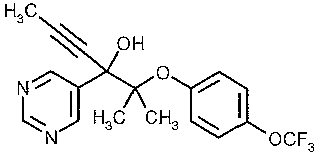 | 500 | 89 |
| 17 | 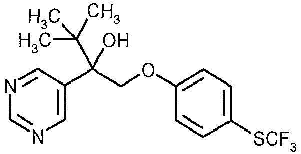 | 500 | 100 |
| 18 | 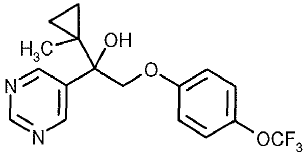 | 500 | 100 |
| 19 | 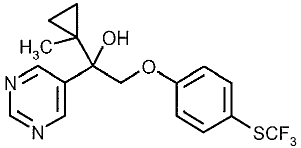 | 500 | 100 |
| 30* | 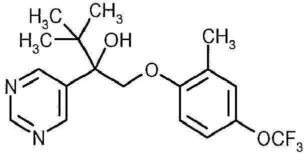 | 500 | 100 |
| 33 | 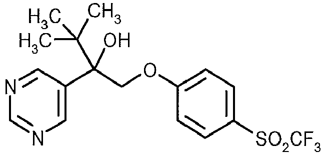 | 500 | 100 |

| | | | |
|---|---|---|---|
| * zum Vergleich | | | |

### Beispiel I: Phakopsora pachyrhizi-Test (Sojabohne) / protektiv

| | | |
|---|---|---|
| Lösungsmittel: | 28,5 | Gewichtsteile Aceton |
| Emulgator: | 1,5 | Gewichtsteile Polyoxyethylenalkylphenylether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit besprüht man junge Pflanzen mit der Wirkstoffzubereitung in der angegebenen Aufwandmenge. 1 Tag nach dem Besprühen werden die Pflanzen mit einer wässrigen Sporensuspension des Erregers von Sojabohnenrost (*Phakopsora pachyrhizi*) inokuliert. Die Pflanzen werden in einem Gewächshaus bei einer Temperatur von ca. 20°C und einer relativen Luftfeuchtigkeit von ca. 80 % aufgestellt. 11 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 100 ppm einen Wirkungsgrad von 80 % oder mehr:

**Tabelle I: Phakopsora pachyrhizi-Test (Sojabohne) / protektiv**

| Nr. | Wirkstoff | Aufwandmenge (ppm) | Wirkungsgrad (%) |
|---|---|---|---|
| 12 | 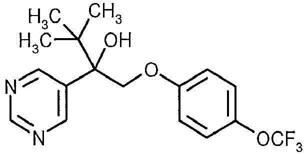 | 100 | 98 |
| 17 | 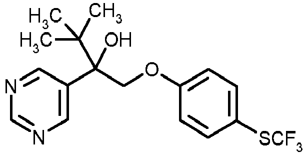 | 100 | 97 |
| 18 | 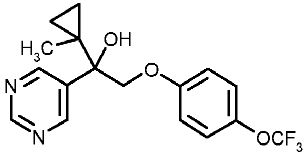 | 100 | 98 |

### Beispiel J: Pyrenophora teres-Test (Gerste) / präventiv

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf präventive Wirksamkeit besprüht man junge Pflanzen mit der Wirkstoffzubereitung in der angegebenen Aufwandmenge. 1 Tag nach dieser Behandlung werden die Pflanzen mit einer wässrigen Sporensuspension von *Pyrenophora teres* inokuliert. Die Pflanzen verbleiben 48 Stunden bei 20°C und 100 % relativer Luftfeuchtigkeit in einer Inkubationskabine. Die Pflanzen werden in einem Gewächshaus bei einer Temperatur von ca. 20°C und einer relativen Luftfeuchtigkeit von ca. 80 % aufgestellt. 7-9 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle I: Phakopsora pachyrhizi-Test (Sojabohne) / protektiv**

| Nr. | Wirkstoff | Aufwandmenge (ppm) | Wirkungsgrad (%) |
|---|---|---|---|
| 2* | 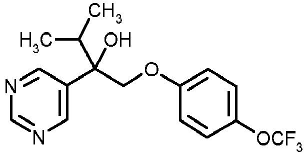 | 500 | 0 |
| | | 100 | 0 |
| 12 | 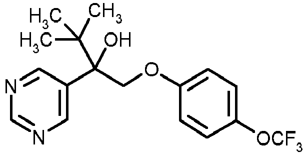 | 500 | 95 |
| | | 100 | 60 |

| | | | |
|---|---|---|---|
| * zum Vergleich | | | |

## Patentansprüche

1. Pyrimidin-Derivate der Formel (I) 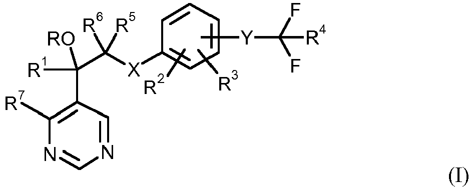 in welcher
X für O, S, SO, SO₂, -CH₂- oder für eine direkte Bindung steht,
R für Wasserstoff, Alkyl, Tri(C₁-C₃-alkyl)silyl, Formyl oder Acetyl steht,
R¹ für *tert-*Butyl, 1-Propinyl, 1-Chlorcyclopropyl, 1-Fluorcyclopropyl, 1-Methylcyclopropyl oder 2,4-Difluorphenyl steht,
R² und R³ jeweils für Wasserstoff stehen,
Y für O, S, SO oder SO₂ steht,
R⁴ für Wasserstoff, Fluor, Chlor oder C₁-C₄-Halogenalkyl steht,
R⁵ und R⁶ gleich oder verschieden sind und jeweils für Wasserstoff, Halogen oder gegebenenfalls substituiertes Alkyl stehen, oder gemeinsam für die Gruppe -CH₂-CH₂- stehen, sodass mit dem Kohlenstoffatom, an welches sie gebunden sind, ein Cyclopropyl-Ring entsteht,
R⁷ für Wasserstoff, Halogen, C₁-C₄-Alkyl oder C₁-C₄-Halogenalkyl steht,
sowie deren agrochemisch wirksamen Salze.

2. Pyrimidin-Derivate der Formel (I) gemäß Anspruch 1, in welcher
X für O, S, CH₂ oder für eine direkte Bindung steht,
R für Wasserstoff, Methyl, Trimethylsilyl, Formyl oder Acetyl steht,
R¹ für *tert*-Butyl, 1-Propinyl, 1-Chlorcyclopropyl, 1-Fluorcyclopropyl, 1-Methylcyclopropyl oder 2,4-Difluorphenyl steht,
R² und R³ jeweils für Wasserstoff stehen,
Y für O oder SO₂ steht,
R⁴ für Wasserstoff, Fluor, Chlor oder C₁-C₂-Halogenalkyl steht,
R⁵ und R⁶ gleich oder verschieden sind und jeweils für Wasserstoff, Fluor, Chlor, Brom, Iod, C₁-C₄-Alkyl oder C₁-C₄-Halogenalkyl, oder gemeinsam für die Gruppe -CH₂-CH₂- stehen,
R⁷ für Wasserstoff, Fluor, Chlor, Brom, C₁-C₄-Alkyl oder C₁-C₂-Halogenalkyl steht.

3. Verfahren zur Bekämpfung von pflanzenpathogenen Schadpilzen, **dadurch gekennzeichnet, dass** man Pyrimidin-Derivate der Formel (I) gemäß Anspruch 1 oder 2, auf die pflanzenpathogenen Schadpilze und/oder deren Lebensraum ausbringt.

4. Mittel zur Bekämpfung von pflanzenpathogenen Schadpilzen, **gekennzeichnet durch** einen Gehalt an mindestens einem von Pyrimidin-Derivaten der Formel (I) gemäß Anspruch 1 oder 2, neben Streckmitteln und/oder oberflächenaktiven Stoffen.

5. Verwendung von Pyrimidin-Derivaten der Formel (I), gemäß Anspruch 1 oder 2 zur Bekämpfung von pflanzenpathogenen Schadpilzen.

6. Verfahren zur Herstellung von Mitteln zur Bekämpfung von pflanzenpathogenen Schadpilzen, **dadurch gekennzeichnet, dass** man Pyrimidin-Derivate der Formel (I) gemäß Anspruch 1 oder 2 mit Streckmitteln und/oder oberflächenaktiven Stoffen vermischt.

7. Verfahren zum Herstellen von Pyrimidin-Derivaten der Formel (I) gemäß Anspruch 1 oder 2, **dadurch gekennzeichnet, dass** man entweder
(A) Oxiran-Derivate der Formel (II) 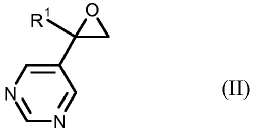 in welcher
R¹ für *tert*-Butyl, 1-Propinyl, 1-Chlorcyclopropyl, 1-Fluorcyclopropyl, 1-Methylcyclopropyl oder 2,4-Difluorphenyl steht,
mit (Thio)phenolen der Formel (III) 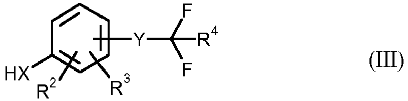 in welcher
X für O, S, SO, SO₂, -CH₂- oder für eine direkte Bindung steht,
R² und R³ jeweils für Wasserstoff stehen,
Y für O, S, SO oder SO₂ steht,
R⁴ für Wasserstoff, Fluor, Chlor oder C₁-C₄-Halogenalkyl steht,
umsetzt und so die Pyrimidin-Derivate der Formel (I-a) 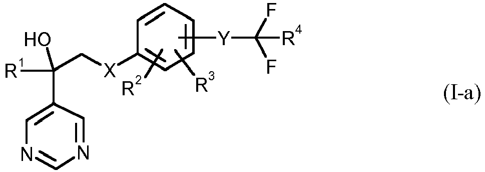 erhält, in welcher X, R¹, R², R³, Y und R⁴ die oben angegebenen Bedeutungen haben,
oder
(B) Ketone der Formel (IV) 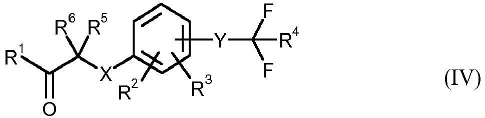 in welcher
X, R¹, R², R³, Y und R⁴ die oben angegebenen Bedeutungen haben
R⁵ und R⁶ gleich oder verschieden sind und jeweils für Wasserstoff, Halogen oder gegebenenfalls substituiertes Alkyl stehen, oder gemeinsam für die Gruppe -CH₂-CH₂- stehen, sodass mit dem Kohlenstoffatom, an welches sie gebunden sind, ein Cyclopropyl-Ring entsteht,
mit Pyrimidinylhalogenide der Formel (V) 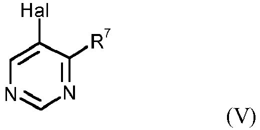 in welcher
R⁷ für Wasserstoff, Halogen, C₁-C₄-Alkyl oder C₁-C₄-Halogenalkyl steht,
Hal für Halogen steht
umsetzt und so die Pyrimidin-Derivate der Formel (I-b) 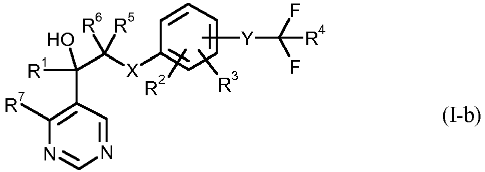 erhält, in welcher X, R¹, R², R³, Y, R⁴ und R⁷ die oben angegebenen Bedeutungen haben,
oder
(C) Bromide der Formel (VI) 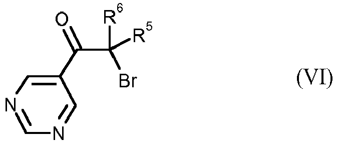 in welcher R⁵ und R⁶ die oben angegebenen Bedeutungen haben,
mit (Thio)phenolen der Formel (III) 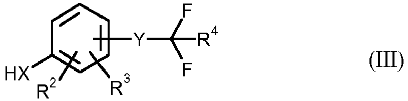 in welcher X, R², R³, Y und R⁴ die oben angegebenen Bedeutungen haben,
umsetzt und so die Ketone der Formel (VII) 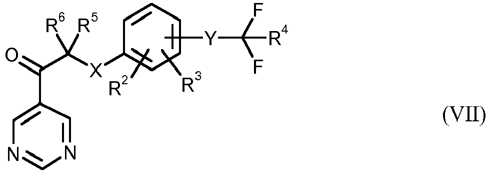 erhält, in welcher X, R², R³, Y, R⁴, R⁵, und R⁶ die oben angegebenen Bedeutungen haben und welche man anschließend mit Organometallverbindungen der Formel (VIII)
R¹-M (VIII)
in welcher
R¹ die oben angegebenen Bedeutungen hat,
M für Metall steht,
umsetzt und so die Pyrimidin-Derivate der Formel (I-c) 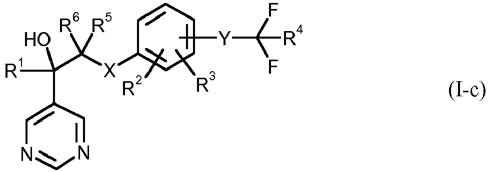 erhält, in welcher X, R¹, R², R³, Y, R⁴, R⁵ und R⁶ die oben angegebenen Bedeutungen haben, oder anschließend
(D) Pyrimidin-Derivate der Formel (I-b) 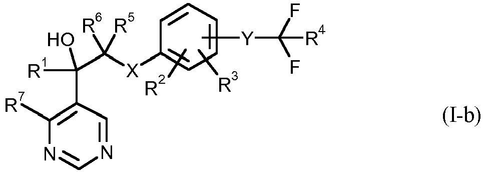 in welcher X, R¹, R², R³, Y, R⁴, R⁵, R⁶ und R⁷ die oben angegebenen Bedeutungen haben, mit Halogeniden der Formel (IX)
R^{a}-Hal¹ (IX)
in welcher
R^{a} für Alkyl, Trimethylsilyl, Formyl oder Acety steht,
Hal¹ für Chlor oder Brom steht,
umsetzt und so die Pyrimidin-Derivate der Formel (I-d) 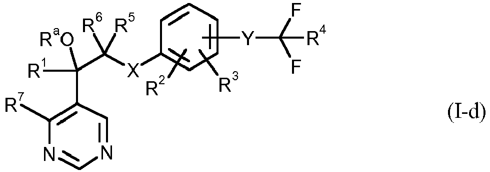 in welcher R^{a}, X, R¹, R², R³, Y, R⁴, R⁵, R⁶ und R⁷ die oben angegebenen Bedeutungen haben, erhält.

8. Oxiran-Derivate der Formel (II-a) 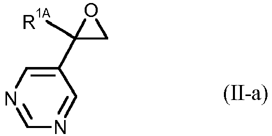 in welcher
R^{1A} für 1-Propinyl oder 2,4-Difluorphenyl steht.

9. Ketone der Formel (IV-a) 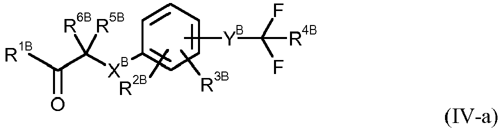 in welcher
X^{B} für O steht,
R^{1B} für *tert*-Butyl, 1-Propinyl, 1-Chlorcyclopropyl, 1-Fluorcyclopropyl oder 1-Methylcyclopropyl steht,
R^{2B} und R^{3B} jeweils für Wasserstoff stehen,
Y^{B} für O, S, SO oder SO₂ steht,
R^{4B} für Wasserstoff, Fluor, Chlor oder C₁-C₄-Halogenalkyl steht,
R^{5B} und R^{6B} gleich oder verschieden sind und jeweils Wasserstoff, Halogen oder gegebenenfalls substituiertes Alkyl stehen, oder gemeinsam für die Gruppe -CH₂-CH₂- stehen, sodass mit dem Kohlenstoffatom, an welches sie gebunden sind, ein Cyclopropyl-Ring entsteht.

10. Ketone der Formel (IV-b) 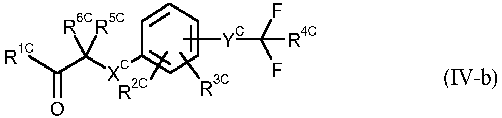 in welcher
X^{C} für S, SO, SO₂ steht,
R^{1C} für 1-Propinyl, 1-Chlorcyclopropyl, 1-Fluorcyclopropyl oder 1-Methylcyclopropyl steht,
R^{2C} und R^{3C} jeweils für Wasserstoff stehen,
Y^{C} für O, S, SO oder SO₂ steht,
R^{4C} für Wasserstoff, Fluor, Chlor oder C₁-C₄-Halogenalkyl steht,
R^{5C} und R^{6C} gleich oder verschieden sind und jeweils Wasserstoff, Halogen oder gegebenenfalls substituiertes Alkyl stehen, oder gemeinsam für die Gruppe -CH₂-CH₂- stehen, sodass mit dem Kohlenstoffatom, an welches sie gebunden sind, ein Cyclopropyl-Ring entsteht.

11. Bromide der Formel (VI-a) 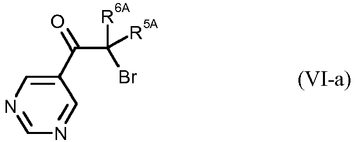 in welcher
R^{5A} für Halogen oder substituiertes Alkyl steht,
R^{6A} für Halogen oder substituiertes Alkyl steht.

## Claims

1. Pyrimidine derivatives of the formula (I) 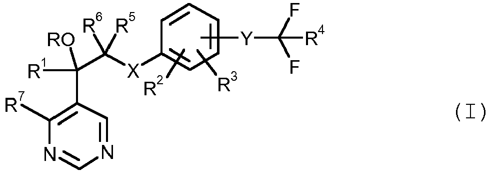 in which
X represents O, S, SO, SO₂, -CH₂- or represents a direct bond,
R represents hydrogen, alkyl, tri(C₁-C₃-alkyl)silyl, formyl or acetyl,
R¹ represents *tert*-butyl, 1-propynyl, 1-chlorocyclopropyl, 1-fluorocyclopropyl, 1 methylcyclopropyl or 2,4-difluorophenyl,
R² and R³ represent in each case hydrogen,
Y represents O, S, SO or SO₂,
R⁴ represents hydrogen, fluorine, chlorine or C₁-C₄-haloalkyl,
R⁵ and R⁶ are identical or different and represent in each case hydrogen, halogen or optionally substituted alkyl, or together represent the group -CH₂-CH₂- such that, together with the carbon atom to which they are attached, a cyclopropyl ring is formed,
R⁷ represents hydrogen, halogen, C₁-C₄-alkyl or C₁-C₄-haloalkyl,
and their agrochemically active salts.

2. Pyrimidine derivatives of the formula (I) according to Claim 1 in which
X represents O, S, CH₂ or represents a direct bond,
R represents hydrogen, methyl, trimethylsilyl, formyl or acetyl,
R¹ represents *tert*-butyl, 1-propynyl, 1-chlorocyclopropyl, 1-fluorocyclopropyl, 1-methylcyclopropyl or 2,4-difluorophenyl,
R² and R³ each represent hydrogen,
Y represents O or SO₂,
R⁴ represents hydrogen, fluorine, chlorine or C₁-C₂-haloalkyl,
R⁵ and R⁶ are identical or different and each repre-sent hydrogen, fluorine, chlorine, bromine, iodine, C₁-C₄-alkyl or C₁-C₄-haloalkyl, or to-gether represent the group -CH₂-CH₂-,
R⁷ represents hydrogen, fluorine, chlorine, bro-mine, C₁-C₄-alkyl or C₁-C₂-haloalkyl.

3. Method for controlling phytopathogenic harmful fungi, **characterized in that** pyrimidine derivatives of the formula (I) according to Claim 1 or 2 are applied to the phytopathogenic harmful fungi and/or their habitat.

4. Composition for controlling phytopathogenic harmful fungi, **characterized in that** it comprises at least one pyrimidine derivative of the formula (I) according to Claim 1 or 2, in addition to extenders and/or surfactants.

5. Use of pyrimidine derivatives of the formula (I) according to Claim 1 or 2 for controlling phytopathogenic harmful fungi.

6. Process for preparing compositions for controlling phytopathogenic harmful fungi, **characterized in that** pyrimidine derivatives of the formula (I) according to Claim 1 or 2 are mixed with extenders and/or surfactants.

7. Process for preparing pyrimidine derivatives of the formula (I) according to Claim 1 or 2, **characterized in that** either
(A) oxirane derivatives of the formula (II) 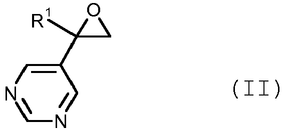 in which
R¹ represents *tert*-butyl, 1-propynyl, 1-chlorocyclopropyl, 1-fluorocyclopropyl, 1 methylcyclopropyl or 2,4-difluorophenyl,
are reacted with (thio)phenols of the formula (III) 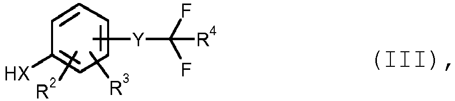 in which
X represents O, S, SO, SO₂, -CH₂- or represents a direct bond,
R² and R³ represent in each case hydrogen,
Y represents O, S, SO or SO₂,
R⁴ represents hydrogen, fluorine, chlorine or C₁-C₄-haloalkyl,
thus giving the pyrimidine derivatives of the formula (I-a) 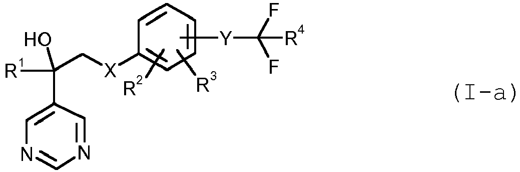 in which X, R¹, R², R³, Y and R⁴ have the meanings given above,
or
(B) ketones of the formula (IV) 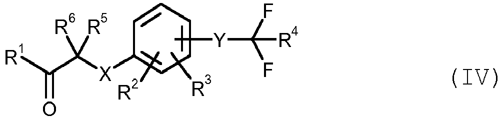 in which
X, R¹, R², R³, Y and R⁴ have the meanings given above,
R⁵ and R⁶ are identical or different and represent in each case hydrogen, halogen or optionally substituted alkyl, or together represent the group -CH₂-CH₂- such that, together with the carbon atom to which they are attached, a cyclopropyl ring is formed,
are reacted with pyrimidinyl halides of the formula (V) 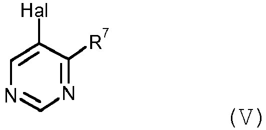 in which
R⁷ represents hydrogen, halogen, C₁-C₄-alkyl or C₁-C₄-haloalkyl,
Hal represents halogen,
thus giving the pyrimidine derivatives of the formula (I-b) 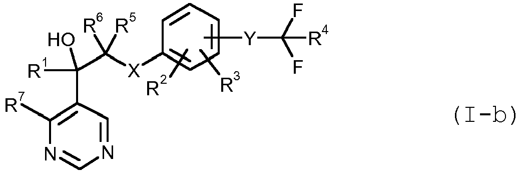 in which X, R¹, R², R³, Y, R⁴ and R⁷ have the meanings given above,
or
(C) bromides of the formula (VI) 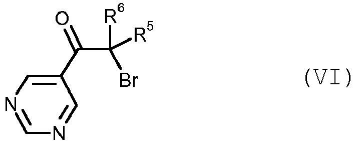 in which R⁵ and R⁶ have the meanings given above,
are reacted with (thio)phenols of the formula (III) 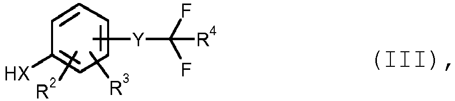 in which X, R², R³, Y and R⁴ have the meanings given above,
thus giving the ketones of the formula (VII) 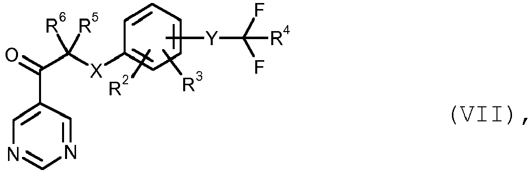 in which X, R², R³, Y, R⁴, R⁵ and R⁶ have the meanings given above, which are then reacted with organometal compounds of the formula (VIII)
R¹-M (VIII)
in which
R¹ has the meanings given above,
M represents metal,
thus giving the pyrimidine derivatives of the formula (I-c) 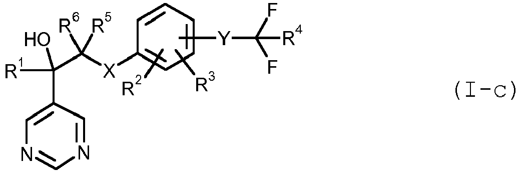 in which X, R¹, R², R³, Y, R⁴, R⁵ and R⁶ have the meanings given above,
or subsequently
(D) pyrimidine derivatives of the formula (I-b) 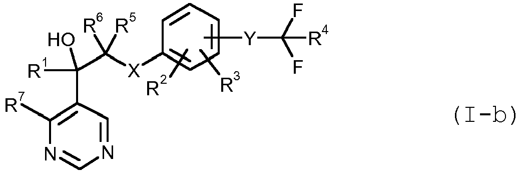 in which X, R¹, R², R³, Y, R⁴, R⁵, R⁶ and R⁷ have the meanings given above,
are reacted with halides of the formula (IX)
R^{a}-Hal¹ (IX),
in which
R^{a} represents alkyl, trimethylsilyl, formyl or acetyl,
Hal¹ represents chlorine or bromine,
thus giving the pyrimidine derivatives of the formula (I-d) 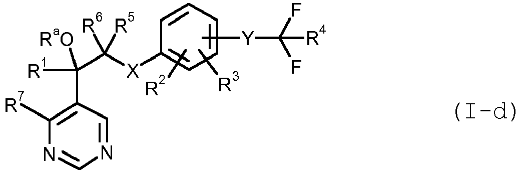 in which R^{a}, X, R¹, R², R³, Y, R⁴, R⁵, R⁶ and R⁷ have the meanings given above.

8. Oxirane derivatives of the formula (II-a) 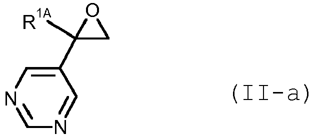 in which
R^{1A} represents 1-propynyl or 2,4-difluorophenyl.

9. Ketones of the formula (IV-a) 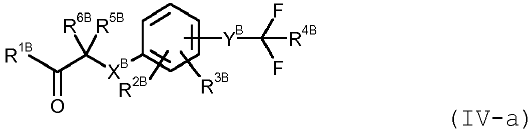 in which
X^{B} represents O,
R^{1B} represents *tert*-butyl, 1-propynyl, 1-chlorocyclopropyl, 1-fluorocyclopropyl or 1-methylcyclopropyl,
R^{2B} and R^{3B} represent in each case hydrogen,
Y^{B} represents O, S, SO or SO₂,
R^{4B} represents hydrogen, fluorine, chlorine or C₁-C₄-haloalkyl,
R^{5B} and R^{6B} are identical or different and represent in each case hydrogen, halogen or optionally substituted alkyl, or together represent the group -CH₂-CH₂- such that, together with the carbon atom to which they are attached, a cyclopropyl ring is formed.

10. Ketones of the formula (IV-b) 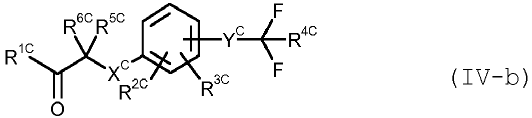 in which
X^{C} represents S, SO, SO₂,
R^{1C} represents 1-propynyl, 1-chlorocyclopropyl, 1-fluorocyclopropyl or 1-methylcyclopropyl,
R^{2C} and R^{3C} represent in each case hydrogen,
Y^{C} represents O, S, SO or SO₂,
R^{4C} represents hydrogen, fluorine, chlorine or C₁-C₄-haloalkyl,
R^{5C} and R^{6C} are identical or different and represent in each case hydrogen, halogen or optionally substituted alkyl, or together represent the group -CH₂-CH₂- such that, together with the carbon atom to which they are attached, a cyclopropyl ring is formed,

11. Bromides of the formula (VI-a) 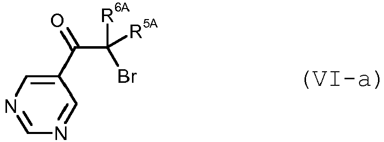 in which
R^{5A} represents halogen or substituted alkyl,
R^{6A} represents halogen or substituted alkyl.

## Revendications

1. Dérivés de pyrimidine de formule (I) 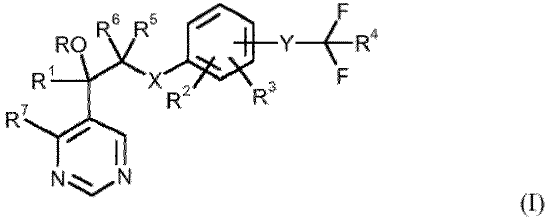 dans laquelle
X représente O, S, SO, SO₂, -CH₂- ou une liaison directe,
R représente hydrogène, alkyle, tri(alkyle en C₁-C₃)silyle, formyle ou acétyle,
R¹ représente *tert*-butyle, 1-propynyle, 1-chlorocyclopropyle, 1-fluorocyclopropyle, 1-méthylcyclopropyle ou 2,4-difluorophényle,
R² et R³ représentent chacun hydrogène,
Y représente O, S, SO ou SO₂,
R⁴ représente hydrogène, fluor, chlore ou halogénoalkyle en C₁-C₄,
R⁵ et R⁶ sont identiques ou différents, et représentent chacun hydrogène, halogène ou alkyle éventuellement substitué, ou représentent ensemble le groupe -CH₂-CH₂-, de manière à former un cycle cyclopropyle avec l'atome de carbone auquel ils sont reliés,
R⁷ représente hydrogène, halogène, alkyle en C₁-C₄ ou halogénoalkyle en C₁-C₄,
ainsi que leurs sels agrochimiquement actifs.

2. Dérivés de pyrimidine de formule (I) selon la revendication 1, dans lesquels
X représente O, S, CH₂ ou une liaison directe,
R représente hydrogène, méthyle, triméthylsilyle, formyle ou acétyle,
R¹ représente *tert*-butyle, 1-propynyle, 1-chlorocyclopropyle, 1-fluorocyclopropyle, 1-méthylcyclopropyle ou 2,4-difluorophényle,
R² et R³ représentent chacun hydrogène,
Y représente O ou SO₂,
R⁴ représente hydrogène, fluor, chlore ou halogénoalkyle en C₁-C₂,
R⁵ et R⁶ sont identiques ou différents, et représentent chacun hydrogène, fluor, chlore, brome, iode, alkyle en C₁-C₄ ou halogénoalkyle en C₁-C₄, ou représentent ensemble le groupe -CH₂-CH₂-,
R⁷ représente hydrogène, fluor, chlore, brome, alkyle en C₁-C₄ ou halogénoalkyle en C₁-C₂.

3. Procédé de lutte contre des champignons phytopathogènes, **caractérisé en ce que** des dérivés de pyrimidine de formule (I) selon la revendication 1 ou 2 sont appliqués sur les champignons phytopathogènes et/ou leur habitat.

4. Agent de lutte contre des champignons phytopathogènes, **caractérisé par** une teneur en au moins un dérivé de pyrimidine de formule (I) selon la revendication 1 ou 2, en plus d'extendeurs et/ou de substances tensioactives.

5. Utilisation de dérivés de pyrimidine de formule (I) selon la revendication 1 ou 2 pour lutter contre des champignons phytopathogènes.

6. Procédé de fabrication d'agents de lutte contre des champignons phytopathogènes, **caractérisé en ce que** des dérivés de pyrimidine de formule (I) selon la revendication 1 ou 2 sont mélangés avec des extendeurs et/ou des substances tensioactives.

7. Procédé de fabrication de dérivés de pyrimidine de formule (I) selon la revendication 1 ou 2, **caractérisé en ce que** soit
(A) des dérivés d'oxirane de formule (II) 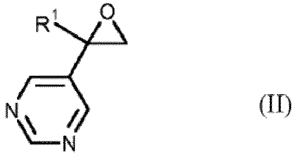 dans laquelle
R¹ représente *tert*-butyle, 1-propynyle, 1-chlorocyclopropyle, 1-fluorocyclopropyle, 1-méthylcyclopropyle ou 2,4-difluorophényle,
sont mis en réaction avec des (thio)phénols de formule (III) 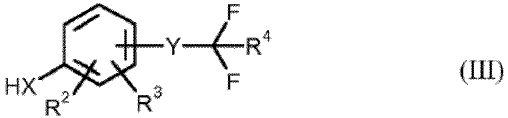 dans laquelle
X représente O, S, SO, SO₂, -CH₂- ou une liaison directe,
R² et R³ représentent chacun hydrogène,
Y représente O, S, SO ou SO₂,
R⁴ représente hydrogène, fluor, chlore ou halogénoalkyle en C₁-C₄,
et les dérivés de pyrimidine de formule (I-a) 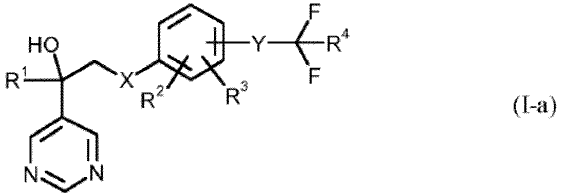 sont ainsi obtenus, dans laquelle X, R¹, R², R³, Y et R⁴ ont les significations indiquées précédemment,
soit
(B) des cétones de formule (IV) 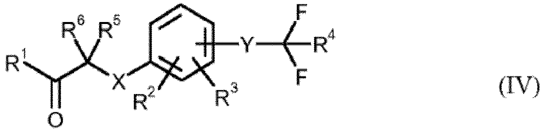 dans laquelle
X, R¹, R², R³, Y et R⁴ ont les significations indiquées précédemment,
R⁵ et R⁶ sont identiques ou différents, et représentent chacun hydrogène, halogène ou alkyle éventuellement substitué, ou représentent ensemble le groupe -CH₂-CH₂-, de manière à former un cycle cyclopropyle avec l'atome de carbone auquel ils sont reliés,
sont mises en réaction avec des halogénures de pyrimidinyle de formule (V) 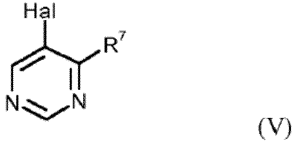 dans laquelle
R⁷ représente hydrogène, halogène, alkyle en C₁-C₄ ou halogénoalkyle en C₁-C₄,
Hal représente halogène,
et les dérivés de pyrimidine de formule (I-b) 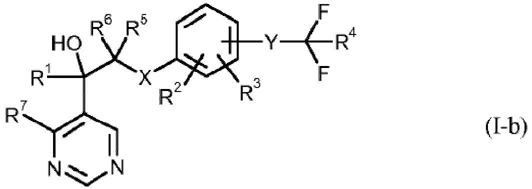 sont ainsi obtenus, dans laquelle X, R¹, R², R³, Y, R⁴ et R⁷ ont les significations indiquées précédemment,
soit
(C) des bromures de formule (VI) 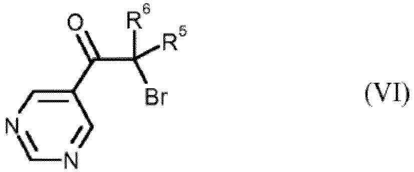 dans laquelle R⁵ et R⁶ ont les significations indiquées précédemment,
sont mis en réaction avec des (thio) phénols de formule (III) 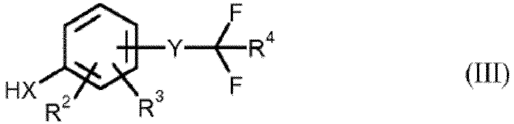 dans laquelle X, R², R³, Y et R⁴ ont les significations indiquées précédemment,
et les cétones de formule (VII) 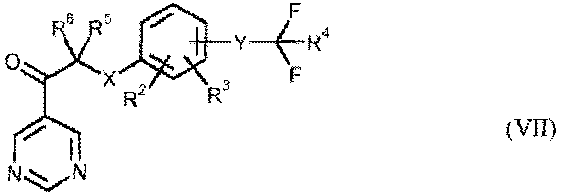 sont ainsi obtenues, dans laquelle X, R², R³, Y, R⁴, R⁵ et R⁶ ont les significations indiquées précédemment, et qui sont ensuite mises en réaction avec des composés organométalliques de formule (VIII)
R¹-M (VIII)
dans laquelle
R¹ a les significations indiquées précédemment,
M représente un métal,
et les dérivés de pyrimidine de formule (I-c) 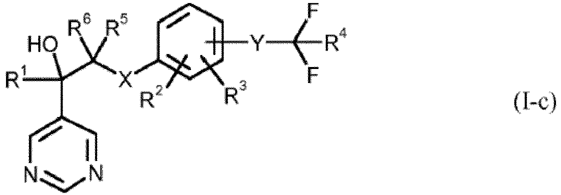 sont ainsi obtenus, dans laquelle X, R¹, R², R³, Y, R⁴, R⁵ et R⁶ ont les significations indiquées précédemment,
soit ensuite
(D) des dérivés de pyrimidine de formule (I-b) 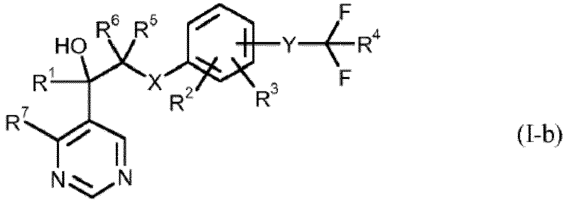 dans laquelle X, R¹, R², R³, Y, R⁴, R⁵, R⁶ et R⁷ ont les significations indiquées précédemment,
sont mis en réaction avec des halogénures de formule (IX)
R^{a}-Hal¹ (IX)
dans laquelle
R^{a} représente alkyle, triméthylsilyle, formyle ou acétyle,
Hal¹ représente chlore ou brome,
et les dérivés de pyrimidine de formule (I-d) 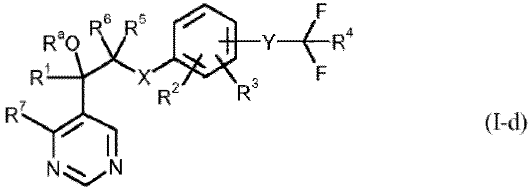 sont ainsi obtenus, dans laquelle R^{a}, X, R¹, R², R³, Y, R⁴, R⁵, R⁶ et R⁷ ont les significations indiquées précédemment.

8. Dérivés d'oxirane de formule (II-a) 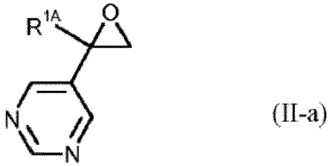 dans laquelle
R^{1A} représente 1-propynyle ou 2,4-difluorophényle.

9. Cétones de formule (IV-a) 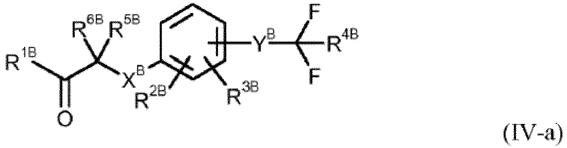 dans laquelle
X^{B} représente O,
R^{1B} représente *tert*-butyle, 1-propynyle, 1-chlorocyclopropyle, 1-fluorocyclopropyle ou 1-méthylcyclopropyle,
R^{2B} et R^{3B} représentent chacun hydrogène,
Y^{B} représente O, S, SO ou SO₂,
R^{4B} représente hydrogène, fluor, chlore ou halogénoalkyle en C₁-C₄,
R^{5B} et R^{6B} sont identiques ou différents, et représentent chacun hydrogène, halogène ou alkyle éventuellement substitué, ou représentent ensemble le groupe -CH₂-CH₂-, de manière à former un cycle cyclopropyle avec l'atome de carbone auquel ils sont reliés.

10. Cétones de formule (IV-b) 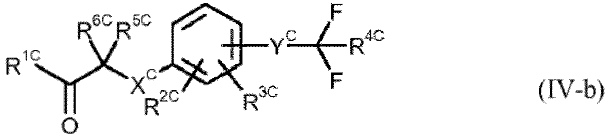 dans laquelle
X^{C} représente S, SO, SO₂,
R^{1C} représente 1-propynyle, 1-chlorocyclopropyle, 1-fluorocyclopropyle ou 1-méthylcyclopropyle,
R^{2C} et R^{3C} représentent chacun hydrogène,
Y^{C} représente O, S, SO ou SO₂,
R^{4C} représente hydrogène, fluor, chlore ou halogénoalkyle en C₁-C₄,
R^{5C} et R^{6C} sont identiques ou différents, et représentent chacun hydrogène, halogène ou alkyle éventuellement substitué, ou représentent ensemble le groupe -CH₂-CH₂-, de manière à former un cycle cyclopropyle avec l'atome de carbone auquel ils sont reliés.

11. Bromures de formule (VI-a) 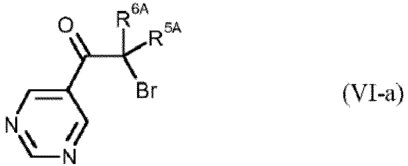 dans laquelle
R^{5A} représente halogène ou alkyle substitué,
R^{6A} représente halogène ou alkyle substitué.
